(19) Europäisches Patentamt / European Patent Office / Office européen des brevets

(11) **EP 4 271 471 B1**

(12) **EUROPEAN PATENT SPECIFICATION**

(45) Date of publication and mention
of the grant of the patent:
**13.08.2025  Bulletin 2025/33**

(21) Application number: **22731257.6**

(22) Date of filing: **07.06.2022**

(51) International Patent Classification (IPC):
**A61N 5/06** (2006.01)     **A61F 9/013** (2006.01)

(52) Cooperative Patent Classification (CPC):
**A61N 5/0618;** A61F 9/00; A61N 5/0622;
A61N 2005/0642; A61N 2005/0663

(86) International application number:
**PCT/EP2022/065326**

(87) International publication number:
**WO 2022/258572 (15.12.2022 Gazette 2022/50)**

(54) **APPARATUS FOR SELECTIVE APPLICATION OF STIMULUS LIGHT**

VORRICHTUNG ZUR SELEKTIVEN ANWENDUNG VON STIMULUSLICHT

APPAREIL D'APPLICATION SÉLECTIVE DE LUMIÈRE DE STIMULUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.06.2021  LU 500257**
**04.02.2022  GB 202201459**

(43) Date of publication of application:
**08.11.2023  Bulletin 2023/45**

(73) Proprietor: **Dopavision GmbH**
**10117 Berlin (DE)**

(72) Inventors:
 • **BAHMANI, Hamed**
   **10117 Berlin (DE)**
 • **SESHADRI, Yeshwanth**
   **10117 Berlin (DE)**
 • **SCHILLING, Tim**
   **10117 Berlin (DE)**
 • **BALSER, Andreas**
   **10117 Berlin (DE)**

(74) Representative: **Synergy IP Group AG**
   **Leonhardsgraben 52**
   **Postfach**
   **4001 Basel (CH)**

(56) References cited:
   WO-A1-2018/212819     WO-A1-2018/224671
   DE-A1- 102011 051 741     US-A1- 2017 000 330

**Description**

Field of the Invention

The invention relates to a device for selective application of stimulus light to an optic nerve head of an eye of a user, as defined in the claims.

Background of the Invention

The 24-hour light-dark (LD) cycle is a fundamental characteristic of the Earth's environment. Behavior and physiology of animals and humans are affected by, and have adapted to, the LD cycle. Most biochemical, physiological, and behavioral variables in humans oscillate according to the LD cycle. These oscillations are termed "circadian rhythms" and are brought about by a circadian timing system of a body. This circadian timing system enables the body to predict the onset of dawn and dusk and adjust physiological and behavioral systems of the body accordingly. It is now established that these circadian rhythms are temporally organized by a circadian clock which maintains temporal synchronization between the body and the external environment, as well as the internal coordination of diverse physiological processes over time.

The eyes of the body provide a sensory system for input of such light-dark time cue signals for synchronizing the LD cycle with the body's circadian rhythm. Light received by the eyes' retina is further processed by the body's brain to synchronize the circadian rhythm. In mammals, a tract of nerves, referred to as the retinohypothalamic tract (RHT), carries information about the light-dark environment directly from the retina via the optic disk and through the optic nerve to the suprachiasmatic nuclei (SCN). The SCN are a cluster of cells in the hypothalamus which receives the transduced light-dark time cue signals, indicating the transition from light to dark, via the RHT from the retinal ganglion cells (RGCs). The SCN cluster distributes the light-dark time cue signals via endocrine and neural pathways to various systems of the body to ensure the various systems are kept synchronous with day and night. When these pathways are disrupted, the rest-activity cycle of the body fails to be synchronized to the LD cycle.

It is known that off-phase light cues may interrupt the normal circadian rhythm. For example, exposure to light late in the biological day, around dusk, will delay the onset of activity in a nocturnal animal, and delay the onset of inactivity in a diurnal animal. Light exposure early in the biological day (dawn) will advance the onset of activity in a diurnal species and advance the onset of sleep in a nocturnal species. Many physiological functions of the body are affected when the light arriving to the eye is off-phase. Moreover, undesired artificial light breaks the natural LD cycle. Light therapy has been shown to be effective for re-tuning the LD cycle. Light therapy (also called phototherapy) consists of exposure to light, daylight, or artificial light, with a specific spectrum and/or with a specific light radiance, for a prescribed amount of time and, in some cases, at a specific time of day.

Originally, scientists held a tacit belief that the light effects on the circadian rhythms, as well as other non-image forming or non-visual effects, were mediated by the classical photoreceptors that mediate vision. This view was shattered when non-image forming responses were demonstrated in mice devoid of the then known "classical" photoreceptors. It was found that light still elicited circadian phase-shifting responses and that the hormone melatonin was suppressed.

Melatonin is the principal hormone of the pineal gland, and is known to mediate many biological functions, particularly the timing of those physiological functions that are controlled by the duration of light and darkness. Light-induced suppression of melatonin had previously been shown to persist in some visually blind people. These data, as well as the demonstration that the spectral sensitivity of non-image forming responses differed from visual responses also in humans, were consistent with the existence of a novel photoreceptive system, subsequently identified as melanopsin.

The photopigment melanopsin is found in the inner retina of humans and other animals and is expressed in particular in a subclass of ganglion cells, called intrinsically photosensitive retinal ganglion cells (ipRGCs). In addition to rods and cones, melanopsin-containing ipRGCs are the third type of retinal cell capable of phototransduction. The ipRGCs respond, in reaction to incoming light, directly via melanopsin, as well as indirectly through signals from rods and cones. It is known that melanopsin is sensitive mainly to short wavelengths and, amongst others, blue light. However, melanopsin is also sensitive to other wavelengths of light in the visible spectrum. The non-image forming or non-visual photo-response of melanopsin to light brings about circadian entrainment in many physiological or body functions. These functions include sleep/wake state (melatonin synthesis), pupil light reflex for regulation of retinal illumination, cognitive performance, mood, locomotor activity, memory, body temperature, etc. The ipRGCs indirect input via the SCN regulates the light-sensitive suppression of melatonin production in the pineal gland. In mice lacking the gene Opn4, which codes for melanopsin, phase shifts, pupillary constriction, and acute suppression of activity in response to light are all attenuated. Abolition of the rods and cones, as well as the Opn4 gene, abolishes all the known image forming and non-image forming effects, demonstrating that both the classical and novel photoreceptive system contribute to these responses.

The human eye can see wavelengths within a range of about 380 nm to about 780 nm. Within this visible light spectrum, some wavelengths can induce acute or cumulative photo-damage to the eye, while other wavelengths play a role in synchronizing human biological rhythms. Historically light treatments have been applied through the eye via ambient light and/or dedicated task light. Providing therapy through conventional lighting systems does not separate or distinguish between visual effects of the provided light (e.g., the image forming function of light) and non-visual effects of the provided light (e.g., non-image forming functions controlling circadian rhythms), as the light is perceived by both image-forming and non-image-forming receptors.

Prior art

A few patent documents are known that discuss the use of light treatment and apparatus used for this treatment. For example, international patent application No. WO 2016/162554 A1 discloses a head-mounted display device which emits light to the eye through a waveguide for treating light-related disorders. The display device has a controller module which adjusts the wavelength of the light emitted to the eye according to the optimally effective wavelength for ipRGCs. The device in the WO'554 application, however, does not avoid activation of the image forming receptors as the method fails to distinguish between the non-image forming receptors and image-forming light receptors in the eye.

International patent application WO 2010/076706 A1 teaches a more specific approach to deliver light therapy to subjects, but the method of this disclosure is limited to special timeframes in a LD cycle, i.e., during sleep or shortly before going to sleep etc. The disclosed embodiment takes a form of sleep mask.

International Patent Application No WO 2014/172641 (Iridex Corporation) teaches the delivery during retinal surgery of a series of short duration light pulses to ocular tissue at a plurality of target locations with a thermal relaxation time delay to limit the temperature rise of the targeted ocular tissue. There is no teaching in this patent application of any use of the system to target the optical disk.

US Patent 5 923 398 A discloses a more practical approach by introducing peripheral light therapy by interactive light field for non-visual or non-image forming stimulation. The approach takes advantage of the fact that the peripheral retina is less engaged in conscious, i.e., image-forming, vision. The peripheral light therapy impacts less on conscious or image-forming vision. However, the device taught in this patent document does not completely exclude the stimulation of image-forming receptors in the eye as rods and cones are still hit by the interactive light field in off-axis or peripheral photon stimulation.

A device and method for treating the visual system of a human being is known from US 2007/0182928 (Sabel, assigned to Novavision Inc.). The method includes the steps of locating and defining a blind zone of deteriorated vision, i.e., a zone of deteriorated image-forming perception, in a user's visual field. The method further includes defining a treatment area which is located predominantly within the blind zone and subsequently treating the human's visual system by presenting visual stimuli to the human's visual system. The visual stimuli are presented on, for example, a computer screen. It will be noted that the term "blind zone" used in this patent application is not to be equated with the term "blind spot" or "optic disk", which is the point at which ganglion cell axons leave the eye and form the optic nerve. The method disclosed in US 2007/0182928 does not include the selective application of light to the "blind spot" or "optic disk" of a user.

International patent application WO 2016/145064 A1 discloses systems and methods for controlling illumination relative to the circadian function of individuals using eyewear. A method for eliminating the interference of light therapy with the normal daily conscious or image-forming vision is not disclosed.

International patent application WO 2018/224671 illustrates a method and apparatus for the application of light onto the optic disk to stimulate the optic disk; the apparatus comprises one or more light emitting sources and an optical system adapted for delivering the light from the light emitting source(s) to an optic disk. This patent application does not disclose doses of light used for the treatment.

US patent 10,444,505 (assigned to Essilor) is directed to a head-mounted display device comprising a light emitting source; an optical waveguide adapted to collect light emitted from the light emitting source and to guide the collected light to the eye of a wearer when the head mounted display device is being worn by the wearer; and a controller adapted to control the emitted spectrum and/or radiance and/or light level emitted by the light emitting source.

The European patent application EP 3 281 056 A1 (assigned to Essilor) is directed to a head-mounted display device comprising a light emitting source; an optical waveguide adapted to collect light emitted from the light emitting source and to guide the collected light to the eye of a wearer when the head mounted display device is being worn by the wearer; a controller adapted to control the emitted spectrum and/or radiance and/or light level emitted by the light emitting source; wherein incidence angles of the light emitted by the light emitting source and from the optical waveguide are determined such that the illumination of the eye is peripheral; and wherein the controller is configured to provide chronobiological regulation or synchronization and/or affective disorders regulation and/or myopia prevention and/or reduction, and/or epilepsy palliative treatment by controlling the light emitting source to provide emissions between 460 nm and 500 nm with specific spatial and temporal patterns.

US patent 9,283,401 (assigned to Myolite) is directed to an eye-wear borne electromagnetic radiation refractive therapy system that comprises an electromagnetic radiation source that directs its electromagnetic radiation to a desired crystalline lens or retina area of a wearer's eye; wherein the electromagnetic radiation source is configured to vary at least one of: (i) the amplitude of the radiation, (ii) the wavelength or spectral properties of the radiation, (iii) the direction of the radiation, and (iv) the area of the ocular components of the eye which are exposed to the radiation.

It is known that the amount of light applied to the optic disk can affect the treatment and that increasing the exposure of the retina to blue light may have associated adverse side effects. There is therefore a need to design a system and method for providing the right dosage of light, including effective dosing regimen, to stimulate the melanopsin whilst avoiding unnecessary light exposure of the retina.

Clinical background

Myopia is typically characterized by excessive ocular growth that increases the risk of serious, sight-threatening complications in adulthood, including cataract, glaucoma, retinal detachment, and myopic maculopathy. It is widely assumed that the mechanism regulating eye growth and myopia progression is localized within the eye 30 (McFadden & Wildsoet, 2020) Currently, there is no standard treatment for myopia progression, however there is a range of myopia control approaches available, including active spectacles, contact lenses, and pharmacological treatments (Wildsoet et al., 2019).

While topical medication with atropine and various contact lens types, including orthokeratology, have been shown to be effective against myopia progression (Huang et al., 2016), both treatments are accompanied by several risks that should be taken into consideration. Even if applied at low dosages, atropine use is off label and has considerable side effects, such as photosensitivity, poor near visual acuity, and temporary stinging or burning. The side effects of orthokeratology and other contact lenses can include mild blurry vision, mild corneal erosion, corneal staining, lens binding, reduced tear film, and infectious keratitis. Infectious keratitis can lead to corneal scars, which require surgical treatment in 10% of cases.

Studies have also investigated the effect of time outdoors on myopia prevention. Randomized controlled trials in school children have reported a significant reduction in myopia incidence rate among children taking part in outdoor programs (Wildsoet et al., 2019). According to a recent meta-analysis one extra hour of time outdoors per week can reduce the risk of myopia by 2%. While the effect of time outdoors on myopia prevention have yielded significant results, only a weak effect on myopia progression is observed (Huang et al., 2016). It is currently not understood the influence of high illuminance or spectral composition of natural light, which tends to be shifted toward the blue end of the visible light spectrum, has on myopia prevention or development.

On the other hand, light therapy can be bad for myopia treatment if light is presented to a user at the wrong time (e.g., out of sync with circadian rhythms). A problem may occur where children need light treatment to help prevent myopia. Children cannot be relied upon to action the treatment at the right times. The method and apparatus of international patent WO 2018/224671 allows invisible or non-image forming light therapy through the eyes and recommends a routine for an optimal protective effect against myopia.

Summary of the disclosure

A device for selective application of stimulus light to an optic nerve head of one of a left eye and a right eye of a user, as defined in the claims is disclosed. The device comprises at least one light emitting source configured to position emitted stimulus light to impinge onto the optic nerve head based on a determined location of the optic nerve head with respect to the user's gaze; at least one screen configured to fixate the user's gaze by engaging the user with content displayed on the at least one screen; a processor for selecting the stimulus light wherein the emitted stimulus light (66) is configured to stimulate melanopsin and is blue light; wherein the at least one screen (50) is the light emitting source (60) wherein the processor (80) comprises and executes software configured for positioning the blue stimulus light (66) on the screen (50) and for providing content displayed on the at least one screen (50), wherein the content is a game, and the game is displayed in the target area (52) of the screen.

The emitted stimulus light may flicker at a frequency in a frequency range between 6 and 20 Hz.

The stimulus light may have an illuminance of more than 20 melanopic lux, preferably approximately 60 melanopic lux.

The at least one light emitting source may further be configured to position the emitted stimulus light to impinge on one of the left eye and the right eye of the user.

The at least one light source may further be configured to dimension the emitted stimulus light to impinge on a portion of the optic nerve head corresponding in size to 80% of the optic nerve head.

The at least one screen may be arranged normal to the user's gaze.

The at least one screen may be arranged at a constant distance from the left eye and the right eye.

The at least one screen may be configured to display the content within at least one target area of the at least one screen, the at least one target area corresponding to an area having a diameter of 1.0 to 5.0 degrees in a foveal region of the left eye and the right eye when the gaze is fixated on the at least one target area.

The at least one target area may be arranged at the center of the at least one screen.

The at least one target area may be configured to fixate one of the left eye and the right eye of the user.

The device may be or comprise a smartphone.

The device may further comprise a virtual reality headset, wherein the smartphone may be insertable into the virtual reality headset.

The device may be a virtual reality headset.

The virtual reality headset may comprise at least one lens for forming a two-lens system with the eye of the user.

The virtual reality headset may comprise one optical path extending between the at least one screen and the left eye and may comprise another optical path extending between the at least one screen and the right eye.

The left eye and the right eye of the user may be in a primary position.

The device may further comprise a game controller for the user to engage with the content displayed on the at least one screen.

The game controller may further be configured to adjust a position of the stimulus light within the screen during calibration.

The device may further comprise a memory device configured to store data relating to the location of the optic nerve head, the data being obtained from one of user-controlled calibration, input into the device of fundus image data, and population data.

The device may be used in a method of selectively applying stimulus light on an optic nerve head of a user. The method comprises positioning at a position at least one light emitting source; fixating the user's gaze by engaging the user with content shown on at least one screen; emitting with respect to the user's gaze stimulus light by means of the at least one light emitting source such that the stimulus light impinges on the optic nerve head. Said method as such and related embodiments described herein are not per se part of the claimed invention which is as defined in the claims, but may be useful for understanding the invention as claimed.

The locating of the optic nerve head of the user may be with respect to the user's gaze.

The locating of the at least one optic nerve head may comprise one of receiving a result from a user-controlled calibration, receiving an input of fundus image data, and processing population data.

The content may be shown to a single one of the one or more eyes of the user.

The light emitting source may be configured to emit stimulus light over a session of use of the device; the session duration may be at least 1 minute and up to 30 minutes, preferably for 12 to 15 minutes.

The session duration may be performed up to five times a day, preferably up to two or three times a day.

The emitting of the stimulus light may be performed for a stimulus duration of at least 1 minute and up to 20 minutes, preferably for between 8 and 10 minutes.

The emitting of the stimulus light may be interrupted by one or more interstimulus intervals.

The interrupting may occur after 30 to 120 seconds of the emitting of the stimulus light.

The one or more interstimulus intervals may last for at least 15 seconds.

The content shown on the at least one screen may be a video game.

The present disclosure further relates to a use of the device of the present disclosure for the treatment of myopia. Reference to any methods of treatment as such, and as described herein below, do not per se constitute the invention which is as defined in the claims, but may be useful for understanding the invention.

Brief description of the drawings

FIG. 1 shows a device according to an aspect of the disclosure.

FIG. 2 shows a method according to an aspect of the disclosure.

FIG. 3 shows the percent change in b-wave amplitude measured 60 minutes after offset of the blue light stimulus light 66. The stimulus duration is shown in seconds for the three measurement conditions: 10 sec, 60 sec, and 600 sec.

FIG. 4 shows mean pupillary changes (%) to the blue and red stimuli for blind spot, parafovea, and periphery conditions over time (ms). Stimulus onset is at 0 ms.

FIG. 5 shows mean pupillary changes (%) for the blind spot (solid line) and periphery (dotted line) conditions in response to blue light. Stimulus onset is at 0 ms.

FIG. 6 shows the mean and standard error of the mean (SEM) of contrast sensitivity (logCS) for the FrACT (left) and TueCST (right) before stimulation and 20 min after blue light stimulation. Dotted line indicates separation between lower than 2 cpd and higher than 2 cpd.

FIG. 7 shows the mean and standard error of the mean (SEM) of the change in amplitude of the PERG P50-N95 and ERG b-wave relative to baseline at 10 and 20 min after blue light stimulation of the blind spot in myopes and non-myopes.

FIG. 8 shows the mean and standard error of the mean (SEM) of the b-wave amplitude ($\mu V$) at baseline and 10, 20, 30, 40, 50, and 60 min after 1 min of blue light stimulation of the blind spot.

FIG. 9 shows the mean and standard error of the mean (SEM) of the b-wave amplitude ($\mu$V) at baseline and 10, 20, 30, 40, 50, and 60 min after 10 min of blue light stimulation of the blind spot.

FIG. 10 shows the mean and standard error of the mean (SEM) of change in b-wave amplitude (%) relative to baseline after no, 10 s, 1 min, and 10 min of blue light blind spot stimulation averaged over measurements taken at 20, 30, 40, 50, and 60 min after blind spot stimulation.

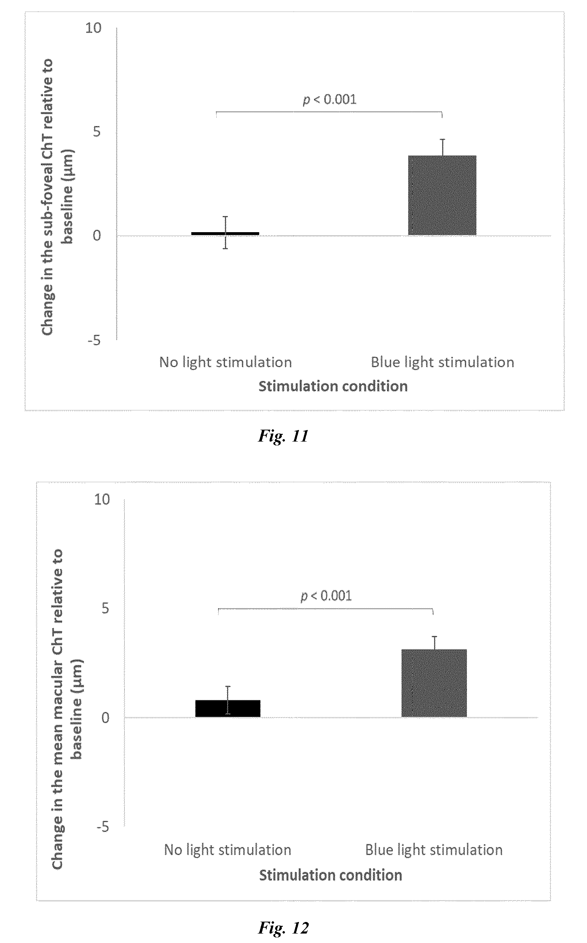

FIG. 11 shows the mean change in sub-foveal choroidal thickness (ChT) relative to baseline ($\mu$m) averaged across all time points and both refractive error groups.

FIG. 12 shows the mean change in macular choroidal thickness (ChT) relative to baseline ($\mu$m) averaged across all time points and both refractive error groups.

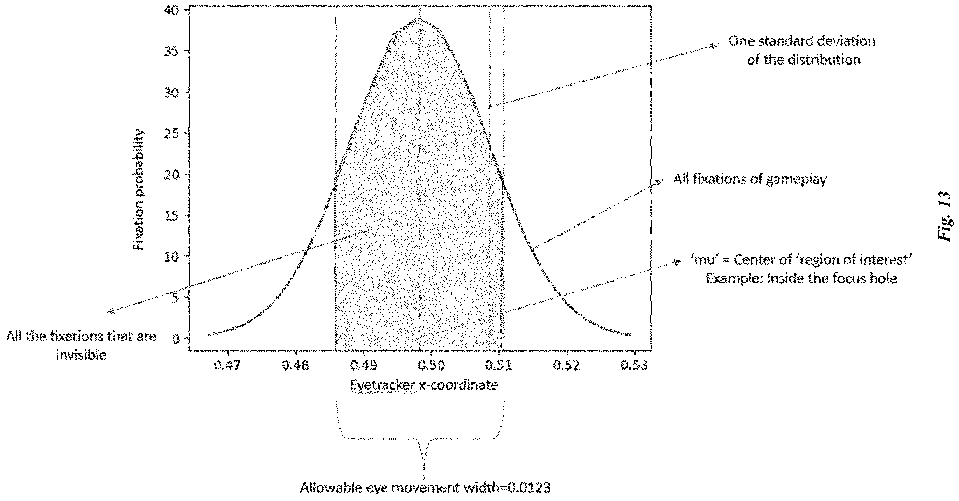

FIG. 13 shows the allowable eye movement range in which the stimulus light is invisible.

Detailed description

Retinal photoreceptors modulate pupil diameter to regulate retinal illumination. The early stages of the pupil light response are formed by both intrinsically photosensitive retinal ganglion cells (ipRGCs) and to a lesser extent rods. It is likely that slower acting melanopsin-containing ipRGCs are the sole contributors to the pupil light response after 1.7 seconds (s) and are responsible for the sluggish recovery of the post illumination pupillary response (PIPR). Melanopsin is sensitive to blue light and is expressed in the cell bodies, dendrites, and proximal axon segments of ipRGCs in rats (Hattar et al., 2002). Melanopsin has an absorption spectrum that peaks at approximately 480 nm, i.e., in the blue range of the visible light spectrum.

The axons of ipRGCs, and those of other retinal ganglion cells, pass through the optic disc or "blind spot" and form part of the optic nerve. The optic disc is also referred to as optic nerve head or as optic disk 36. The optic disc contains no rods or cones. Light falling onto the optic disc or optic nerve head 36 is not consciously perceived, i.e., does not lead to image-forming perception. It is not fully understood whether the presence of melanopsin in the axons of ipRGCs makes the optic disc, the optic disc or optic nerve head 36 sensitive to blue light.

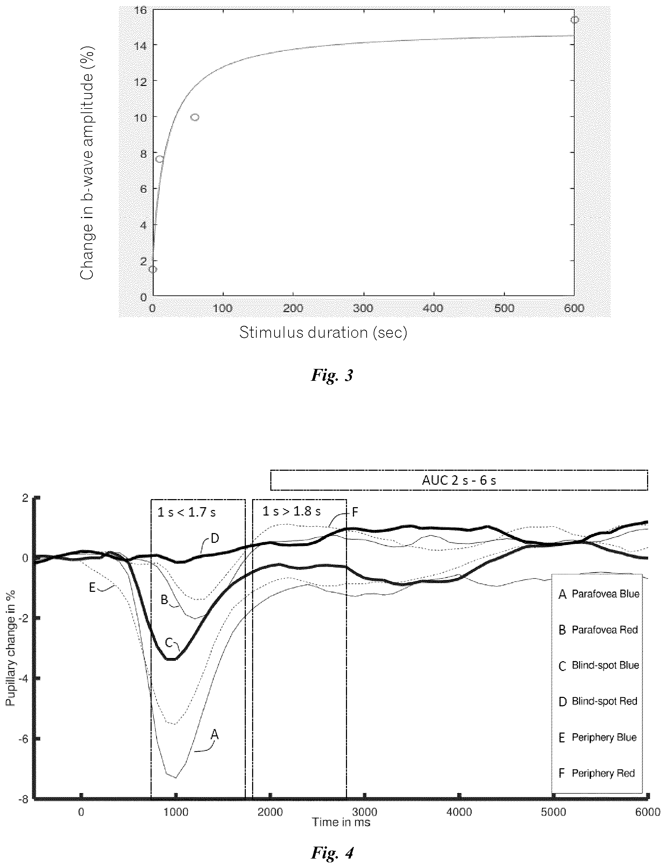

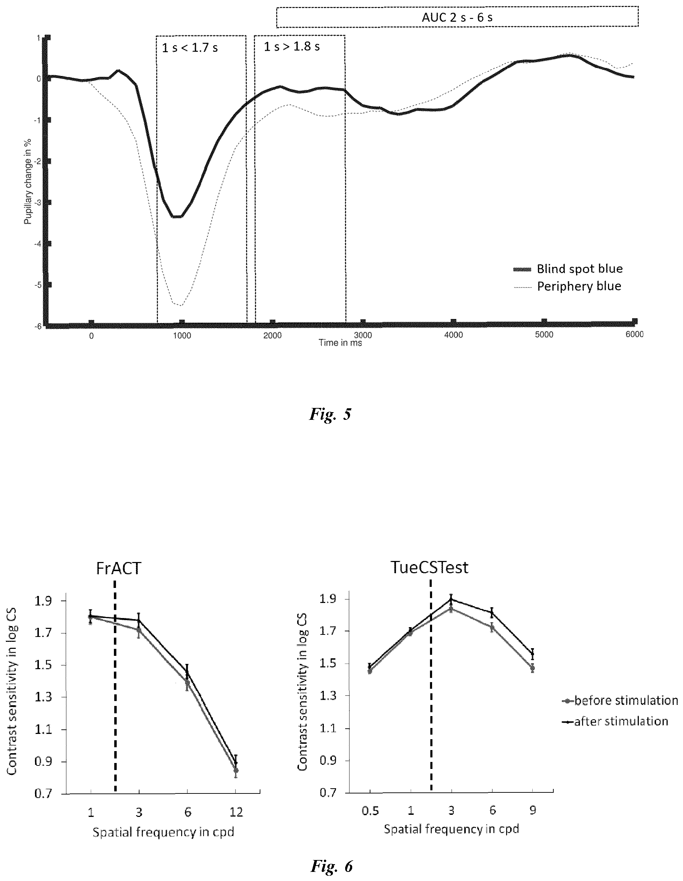

FIG. 4 shows mean and standard error of the mean (SEM) of pupillary change (%) to blue and red stimuli for blind spot, parafovea, and periphery conditions over time (ms) with a stimulus onset at 0 ms. FIG. 5 shows mean and standard error of the mean (SEM) of pupillary change (%) for the blind spot (solid line) and periphery (dotted line) conditions in response to blue light with a stimulus onset at 0 ms (Schilling et al., 2020). It was found thus that selective stimulation of the optic disc or optic nerve head 36 of young adults with blue light induced a greater pupillary response (constriction) compared to stimulation with red light. The results are consistent with a presence of melanopsin in the axons of ipRGCs at the optic disc.

The contribution of melanopsin to the pupillary light response in view of the absence of classical photoreceptors, i.e., of rods and cones, in the blind spot or optic disc is not fully understood.

The change in **PIPR** was examined following stimulation of the blind spot, parafovea, and periphery with light.

It is not known whether excitation of melanopsin regulates the retinal dopaminergic system, e.g., through retrograde signaling from ipRGCs to dopaminergic amacrine cells which are capable of releasing dopamine (Zhang et al., 2008), resulting in modulation of dopamine-driven light adaptation and retinal circadian regulation. It is further not known whether dopamine is released after excitation of melanopsin in the optic disc or optic nerve head 36. Dopamine supports a number of functions in the retina and there is evidence that it also contributes to contrast sensitivity. Behavioral studies in healthy adults have found that levodopa and nomifensine, both dopamine agonists, i.e., compounds that activate dopamine receptors, improve contrast sensitivity at medium and high spatial frequencies, in particular those greater than 2 cycles per degree (cpd). Dopamine is also involved in retinal light adaptation.

The sensitivity of the optic disc to blue light potentially results in an increase of retinal dopamine levels. As mentioned before, increased retinal dopamine levels are known to increase contrast sensitivity. FIG. 6 shows mean and standard error of the mean (SEM) of contrast sensitivity (logCS) for the Freiburg Visual Acuity Test (FrACT) and the Tübingen Contrast sensitivity Test (TueCST) before and 20 min after stimulation of the optic nerve head 36 with blue light. Dotted line indicates separation between lower than 2 cpd and higher than 2 cpd. A melanopsin-triggered increase in dopamine has been found upon stimulating the optic nerve head 36 (i.e., the blind spot) with blue light. This increase of dopamine improves contrast sensitivity for stimuli with spatial frequencies higher than 2 cpd.

Both ON pathway (Chakraborty et al., 2015) and dopamine (Feldkaemper & Schaeffel, 2013) abnormalities have been implicated in ocular growth regulation and refractive error development. Studies investigating myopia using ERG, a useful, non-invasive technique to probe the potential retinal mechanisms of myopia development, have reported a reduction in the b-wave amplitude of myopes and an inverse association between the b-wave amplitude and axial eye length. The b-wave is a measure of human retinal function that primarily reflects ON bipolar cell activity. In animal models, experimental myopia has been associated with reduced retinal dopamine levels in a variety of species. Dopamine agonists have been found to suppress the development of experimental myopia. Rearing animals under bright light conditions has a similar inhibitory effect on myopia development. It is not fully understood whether the inhibitory effect of bright light, which in some cases is greater when short-wavelength light is used, is mediated by a light-driven increase in retinal dopamine. Furthermore, children with high myopia are more susceptible to sleep disturbances, potentially due to an abnormality regarding dopamine which is known to be involved in circadian rhythm entrainment. It is not known whether there is some functional change in the myopic retina that is localized in the inner layers and involves the retinal dopaminergic system.

A potential target for dopaminergic modulation in the myopic eye 30 is the intrinsically photosensitive retinal ganglion cells (ipRGCs), the melanopsin-containing axons of which pass through the optic disc 36.

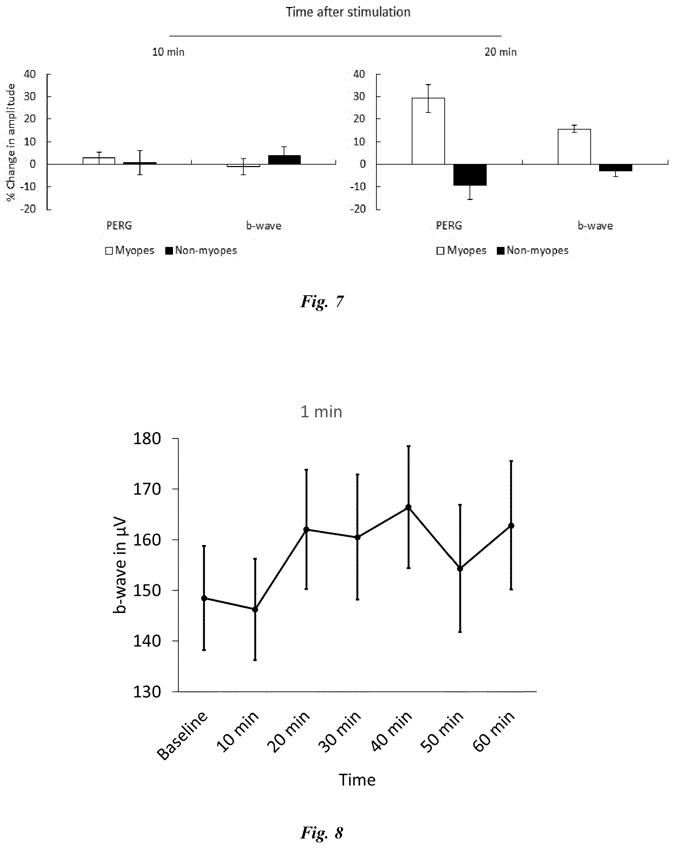

The effect of blue light stimulation of the optic disc or optic nerve head 36 (also termed "blind spot") on the full-field ERG and pattern ERG (PERG) of myopes compared to non-myopes was investigated. It was reported that changes in retinal electrical activity follow stimulation of blind spot melanopsin with blue light. Fig. 7 shows significant changes in the response of myopes but no significant changes in non- myopes. It is not fully understood whether the changes in retinal electrical activity upon stimulation of melanopsin involve retrogradely upregulating dopamine release in the inner plexiform layer as well as dopamine-mediated retinal processes and activity (Amorim-de-Sousa et al., 2020).

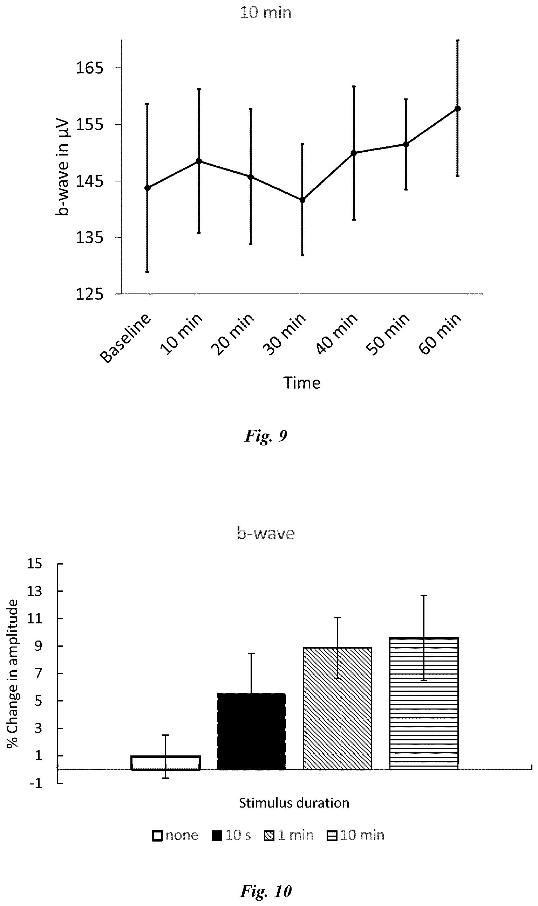

It was further examined how the ERG responds to different durations (i.e., 0s, 10s, 1min, and 10min) of light stimulation over longer periods of time, i.e., at 10min, 20min, 30min, 40min, 50min, and 60min after stimulation (see FIGS. 8-10). It was observed that the b-wave amplitude was increased following all tested stimulation durations relative to no stimulation with greater increases for the 1-minute and 10-minute stimulation durations and a smaller effect of a duration of 10 seconds of stimulation. After 10 minutes of stimulation, the b-wave amplitude increase was not observed until 60 minutes after stimulation of the optic nerve head 36. On the other hand, an increase in b-wave amplitude was measured 20 minutes after stimulation of the optic nerve head 36 for 1 minute. It is not fully understood whether these results mean that varying durations of blue light stimulation of the blind spot elevate ON bipolar cell activity in the retina, which may have the effect of reducing the myopic response.

It is not fully understood whether choroidal thickness changes provide a short-term biomarker of vision-dependent mechanisms that regulate eye growth and precede longer-term changes in eye size. Processes leading to emmetropia, or hyperopia are associated with choroidal thickening, whereas those leading to myopia are accompanied by choroidal thinning. The choroid has been found to thicken in response to increased light exposure and ambient light appears to have a protective effect against excessive eye growth and myopia, which may be mediated by the retinal dopaminergic pathway.

It was explored whether changes in choroidal thickness could be used as a clinical biomarker that represents the intrinsic activity of the melanopsin-driven signaling pathway. It was investigated whether blue light stimulation, as opposed to the absence of light stimulation, of the optic disc causes an increase in choroidal thickness and a decrease in axial length. An optical coherence tomography (OCT) study was conducted to explore whether changes in choroidal thickness could be used as a clinical biomarker that represents the intrinsic activity of the melanopsin-driven signaling pathway. Choroidal thickness was measured before and after young myopic and emmetropic adults underwent blue light stimulation of the optic nerve head 36 for a stimulus duration of one minute ($\lambda_{peak}$ = 450 nm; 15 Hz; 22 cd/m2). Custom-developed software and a Samsung Galaxy S7 inserted into a virtual reality headset were used to deliver the light. The users calibrated the stimulus light 66 to their blind spot location, after which they underwent a 10 min washout period and 5 min of dark adaptation before baseline OCT imaging and optical biometry were performed. Post-stimulation OCT measurements were taken at 0, 10, 20, 30, and 60 mins. Axial length was measured only at 60 min. Rapid and sustained choroidal thickening was measured over a 60-minute period following activation of melanopsin at the optic nerve head 36 using blue light. FIG. 15 shows the time-averaged change in sub-foveal choroidal thickness (ChT). FIG. 16 shows the time-averaged change in macular choroidal thickness. As a short-term biomarker, the increase in choroidal thickness may signal longer-term changes in ocular growth with repeated exposure to the blue light stimulus light 66, a process which could involve the retinal dopaminergic system.

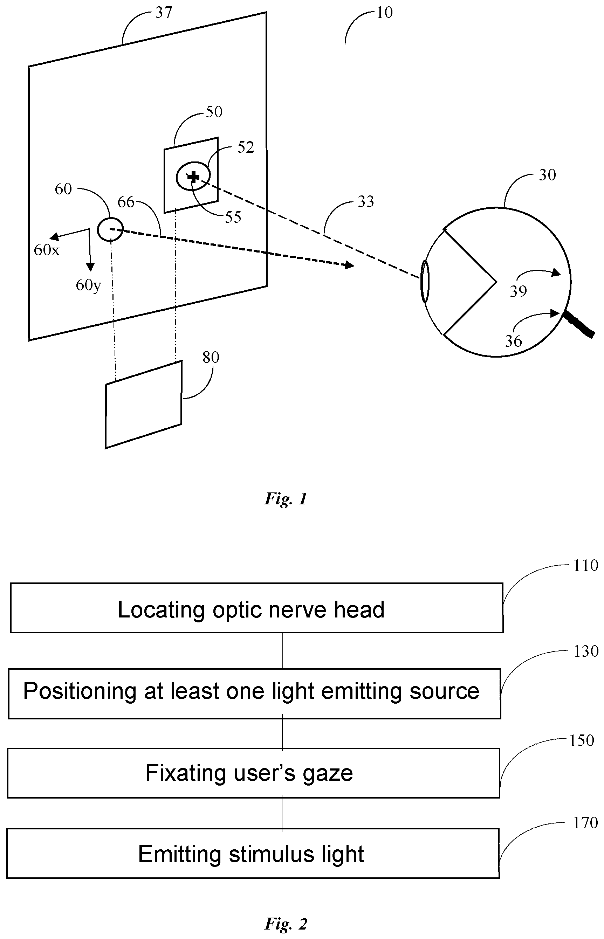

The present disclosure is directed to a device as defined in the claims. The device may be used in a method of applying stimulus light to a user's optic nerve head (FIG. 2). The device comprises a computer program product, such as a software or a software app. The device 10 (FIG. 1) has a processor 80 for executing the computer program product, such as a smart phone or a virtual reality (VR) device, to emit 170 by means of at least one light emitting source 60 and deliver, i.e. emit 170, the blue light stimulation light 66 to the optic nerve head 36 (also known as blind spot). The blue light stimulation light 66 is delivered, i.e., is emitted 170, while the user is provided with content by the software or software app. In one aspect, the content is a game displayed on a screen 50 of the device 10. The user is engaged in gaming. The user keeps the gaze 33 stable and directed to the screen 50. The gaze 33 is fixated 150 on a target area 52 of the screen 50, in which the content, e.g., the game, is displayed. The term "gaze" is to be understood as the user's eye 30 being directed to a point in the user's visual field 37. In this case, the pupil, the fovea 39, and the optic nerve head 36, amongst other parts of the eye 30, are in a defined orientation, e.g., with respect to a line connecting the user's pupil, e.g., the center of the pupil, and the point 55 in the user's visual field 37 at which the user's gaze 33 is directed. In one aspect of the disclosure, the eyes 30 of the user are in a primary position, i.e., directed straight ahead, when the user's gaze 33 is directed at the content with which the user is engaged.

The screen 50 has a position within the visual field 37 of the eye 30. The at least one light emitting source 60 has a position 60x, 60y within the visual field 37 of the eye 30. The position 60x, 60y of the at least one light emitting source 60 may overlap or may not overlap (as is the case shown in the aspect of FIG. 1) with the screen 50. In some aspects of the disclosure, the software or software app may run on commercially available smartphones, e.g., Android smartphones.

The device of the present disclosure may in one aspect relate to a smartphone in conjunction with a VR headset and a suitable game controller. The VR headset enables stimulating both eyes 33 of the user. When using the VR headset, the distance between the screen 50, e.g., of the smartphone or mobile device, and the eye 30 is kept substantially constant, which facilitates calculations to provide the stimulus light with sufficient illuminance and to adjust the illuminance of the shown content. Furthermore, the orientation of the screen 50 may be substantially normal with respect to the direction on the gaze 33 when using the VR headset. Moreover, when using the VR headset, the left eye 30 and the right eye 30 of the user may be provided with the stimulus light and the content individually (as is further explained below).

In one aspect of the present disclosure, the screen 50 may provide the stimulus light and the content individually and separately to the left eye 30 and the right eye 30 of the user. In other words, in this aspect of the disclosure, the content and stimulus light provided to the left eye 30 will not be perceived by or impinging on, the right eye 30. Likewise, the content and stimulus light provided to the right eye 30 will not be perceived by or imping on, the left eye 30 in this aspect of the disclosure.

The content and the stimulus light may be individually provided to the right eye 30 and the left eye 30 by means of the screen 50 as well as a further one of the screens 50. Alternatively, the screen 50 may be split into two portions, e.g., halves, such that one portion of the screen 50 provides the stimulus light and the content to the left eye 30, and the other portion of the screen 50 provides the stimulus light and the content to the right eye 30.

For instance, the stimulus light provided to the left eye 30 may be emitted by the light emitting source 60 positioned in the position 60x, 60y such that the stimulus light impinges on the optic nerve head 36 of the left eye 30. However, the stimulus light will not impinge on the optic nerve head 36 of the right eye 30. Furthermore, the stimulus light provided to the right eye 30 may be emitted by the light emitting source 60 positioned in another one of the positions 60x, 60y such that the stimulus light impinges on the optic nerve head 36 of the right eye 30. However, the stimulus light will not impinge on the optic nerve head 36 of the left eye 30. In this aspect of the disclosure, the device 10 may comprise the light emitting source 60 for, e.g., providing stimulus light to the left eye 30. The device 10 may further comprise a further one of the light sources 60 for, e.g., providing the stimulus light to the right eye 30.

Furthermore, the content may be displayed, e.g., to the left eye 30 of the user within the target area 52, and the content may be displayed, e.g., to right left eye 30 of the user within another one of the target areas 52. In a further aspect of the disclosure, the target area 52 and the further one of the target areas 52 may partially overlap. An overlap of the target area 52 and the further one of the target areas 52 may depend on the distance between the screen 50 and the eyes 30 of the user.

In this aspect of the disclosure, in which the stimulus light and the content are provided individually to the left eye 30 and the right eye 30 of the user, two optical paths are provided. The two optical paths extend between the screen 50 and one of the left eye 30 or the right eye 30 of the user. One of the optical paths enables the stimulus light and the content, e.g., to the left eye 30. The other one of the optical paths provides the stimulus light and the content, e.g., to the right eye 30. In one aspect, the two optical paths may be separated by a barrier shielding one of the left eye 30 and the right eye 30 from the stimulus light and the content provided for the other of the left eye 30 and the right eye 30. In an alternative aspect, the two optical paths may be separated by means of polarization, e.g., using polarizing filters. One polarizing filter or one set of polarizing filters may be used to provide the stimulus light and the content to the left eye 30. Another polarizing filter or another set of polarizing filters may be used to provide the stimulus light and the content to the right eye 30.

The software app delivers the flickering blue light stimulus light 66 to the blind spot 36 while using a game. The game is designed such that the user must constantly look at one particular point or a required visible area 52 on the display 50 or screen 50 to succeed. According to the present disclosure the screen 50 blackens out/darkens outside the required visible area. This required visible area will be referred to as a "focus circle" or "target area" 52. There is almost no observable difference to the user between the game according to the present disclosure, in particular when the VR headset is used for display of the game, and other video games, as the blue light stimulus light 66 is displayed, i.e., impinges, on the user's optic nerve head 36, i.e., is not visually perceptible.

In one aspect of the disclosure, the game may comprise one or more game levels displayed to the user. The user engages with the game by playing the game. The one or more game levels may be played by the user in a consecutive manner. Ones of the one or more games levels may comprise instructions displayed to the user before playing a corresponding one of the one or more levels.

The displaying to the user of the one or more game levels may comprise displaying at least one of a plurality of target icons or game icons. This displaying of the target icons or the game icons may occur for a predetermined period of time. The playing by the user of the game may comprise looking at and memorizing, one or more of the displayed plurality of target icons. The playing by the user of the game may further comprise actuating the game controller, e.g., pressing a button of the game controller, after memorizing one(s) of the plurality of target icons. Alternatively, the user may wait for the game to automatically continue after memorizing the at least one of the plurality of target icons.

The game controller may be a wireless game controller.

The displaying to the user of the game may further comprise changing one or more of the plurality of target icons. The changing may comprise modifying the plurality of target icons or selecting a different one of the plurality of target icons to be displayed. The selecting of different one of the plurality of target icons may be repeated.

The playing by the user of the game may comprise actuating the game controller, e.g., pressing a button of the game controller, upon identification by the user of the memorized one(s) of the plurality of target icons, when the memorized target icons has been selected to be displayed. The playing by the user of the game may further comprise measuring a reaction time needed by the user to identify the memorized one(s) of the plurality of target icons when the memorized target icons has been selected to be displayed.

The playing by the user of the game may further comprise determining a performance score for the user based on the measured reaction time. The determining of a performance score for the user may further comprise determining a correctness associated with an actuation by the user of the game controller, i.e., determining whether the game controller was actuated upon correctly identifying the memorized at least one of the plurality of target icons.

The playing of the game may comprise interrupting the game for the duration of an interstimulus interval. The interstimulus interval may last for 15 seconds. The playing by the user of the game may comprise indicating to the user the beginning of a next one of the one or more game levels. The indicating may comprise presenting a sound to the user. The playing of the game may comprise notifying the user of a last one of the one or more game levels to be played.

In one aspect of the disclosure, the parameters of the treatment may be as follows

A position 60x, 60y of the blue light stimulus light 66 may be such that the blue light stimulus light 66 impinges at the center of the optic disc or optic nerve head 36. The location of the optic disc or optic nerve head 36 may be determined, i.e., the optic disc or optic nerve head 36 may be located 110 in a step of locating 110 the optic nerve head 36, by an ophthalmologist/optometrist, based e.g., on an image of the fundus of the eye 30, i.e., the interior surface of the eye 30 opposite the lens and including the retina, the optic nerve head 36, the macular, the fovea 39, and the posterior pole. Optionally, available information on the location of the optic nerve head 36, which was previously determined may be used. The ophthalmologist/optometrist may input the determined or pre-determined location of the optic nerve head 36 into a stimulus-positioning device, e.g., the device 10, the light emitting device 60, or the screen 50. In one aspect of the present disclosure, the stimulus-positioning device comprises a screen 50 and a processor 80 with data-processing logic, such as a smartphone, that, based on computations by the software app running on the processor, positions 130 the stimulus light 66 on the screen 50.

A shape of the stimulus light 66 may be circular. A size of the circularly shaped stimulus light 66 may have a radius of an angular size of 2.2 deg (visual angle).

The stimulus light 66 may be flickering and have a frequency of, e.g., 15 Hz. The stimulus light 66 may be a rectangular function with a frequency of, e.g., 15 Hz. In another aspect, the frequency at which the stimulus light 66 flickers may be in the range of 6 to 20 Hz.

A color of the stimulus light 66 may be set using the RGB-color code. The color may be set to, e.g., (0, 0, 255).

A brightness or illuminance of the blue light stimulus light 66 is for example at least about 20 melanopic lux for each blue light stimulus light (66). In a further aspect, the brightness may be the maximum brightness that is deliverable by the screen 50 of the smartphone model (i.e., Samsung Galaxy S7), corresponding to emitting 170 a melanopic lux of 60 from each of the blue discs, i.e., to each eye (30).

To keep the user's gaze 33 stable, the content (e.g., a game) is displayed in a target area 52 on the screen 50 corresponding to a portion within the user's retina including the fovea. In other words, when the user gazes 33 at the target area 52 of the screen 50, the content displayed in the target area 52 of the screen 50 is imaged onto a portion of the retina which includes the fovea 39. The content, such as the game, furthermore, involves active engagement of the user. The user's performance is quantified (accuracy and reaction time) as an index for the user's engagement (also referred to as performance score). The fovea 39 is an area on the retina corresponding to the area in the visual field 37, e.g., the target area 52 on the screen 50, that a human eye 30 is fixated on for a clear vision. In other words, while maintaining fixation of gaze 33 on a fixation point, the image of the fixation point projects, or is imaged, onto the fovea 39.

Optionally, the location of the target area 52 relative to the screen 50, and thus the location where the content is displayed, remains constant throughout a user session. For example, the target area 52 may be arranged at the center of the screen 50. Moreover, the size of the displayed content may be relatively small such as to constrain the variability of the user's gaze 33, thereby providing that the blue light stimulus light 66 is directed to the optic nerve head 36. For example, the size of the content may be equivalent to a circle having a radius of 2 deg (visual angle) or less, such as about 1.5 deg.

The device as claimed may be used in a method of treatment; said method as such and embodiments thereof as disclosed herein below is not part of the invention as defined in the claims, but may be useful for understanding the invention. The method enables slowing of the rate of myopia onset and/or progression. In one aspect, the method enables slowing down myopia progression in children. The children may be aged 6 to 14 years old. However, other ages are possible. The method according to the present disclosure is indicated for myopic children with a refractive error between -0.75 and -5.00D with evidence of progression (0.25 D/y). For this purpose, the user performs the method in, for example, at least one session per day. In another aspect, the user performs two sessions per day. Optionally, three or more sessions may be performed per day.

A recommended timing of sessions using the method is as follows. A first session during which the method is applied may occur in the morning before the child user is going to school. A second session may occur when children arrive home from school (possibly early afternoon). In one aspect, the second session occurs at least 2 hours after the first session but no later than 3 hours before sleeping.

The method according to the present disclosure enables increasing retinal dopamine release. The retinal dopamine release enables eye growth regulation. The eye growth regulation is achieved by stimulating the axons of melanopsin-containing ipRGCs at the optic nerve head 36 (or "optic disc") with short-wavelength light in the blue range. The treatment is in one aspect of the disclosure applied using a smartphone inserted in a VR headset.

The method according to the present disclosure enables increasing retinal dopamine levels using blue light stimulation of the optic disc, also termed optic nerve head 36 or blind spot. To minimize any potential influence of blue light on the retina, the method targets the optic disc or optic nerve head 36, in which the axons of intrinsically photosensitive retinal ganglion cells converge and form part of the optic nerve. Stimulating the melanopsin-containing the axons of the ipRGCs in this way potentially increases retinal dopamine activity retrogradely, as mentioned above, the increase in the retinal dopamine potentially initiates a signaling cascade that ultimately slows ocular growth and the myopia progression.

A series of scientific experiments have been conducted to investigate the proposed mechanism of action of the method according to the present disclosure.

Several studies have investigated the risks of blue light regarding user safety. While animal research has demonstrated the potential hazards blue light poses to the retina, these animal studies used light parameters and exposure times leading to considerably higher overall light exposure than the settings of the method according to the present disclosure. Current light-emitting devices, such as the smartphones, are not thought to present any significant acute or subacute risk to the user's retina (Clark et al., 2018). This is particularly true considering that, according to the method of the present disclosure, children are only exposed to the blue light stimulus light 66 for a maximum duration of stimulation of 10 minutes (i.e., the active stimulus duration), twice a day. In comparison, the safe viewing limit for the blue light emitted from a Samsung Galaxy S7 is 28 consecutive hours, according to the IEC 62471:2006 norm (Photobiological safety of lamps and lamp systems).

Other important safety considerations include the potential effects of the blue light on the circadian rhythm and the effect of the temporal modulation (flicker) of the stimulus light 66. The effects of the blue light on the sleep-wake cycle are well documented, however the extent to which the sleep-wake cycle is affected by blue light depends on the time of exposure. The melanopsin-containing ipRGCs are responsible for entraining the circadian rhythm to the solar day and are thought to be most sensitive to the blue light at night. Studies applying the blue light in the evening agree that the evening blue light can significantly impact individuals' sleep-wake cycle and sleep quality. This influence of the blue light has been considered when defining the recommended time of the day to complete the treatment sessions, as well as when the software to implement the method is enabled.

Additionally, to increase the effectiveness of the treatment, the light stimulus light 66 will flicker. It not fully understood how the flicker of the light impacts on the method of the present disclosure. It is acknowledged that flickering stimuli could trigger photosensitive seizures and therefore the treatment may not be suitable for those children diagnosed with, or with a family history of, photosensitive epilepsy or seizure.

Overall, the present disclosure indicates that blue light stimulation may have beneficial effects on the ocular growth, and thus the myopia progression and/or the myopia onset, without posing any significant safety concerns when applied according to the instructions for use.

As mentioned earlier, research has implicated the ipRGCs in a number in intraretinal interactions, including with dopaminergic amacrine cells based on retrograde communication between the ipRGCs and the dopaminergic amacrine cells, which potentially facilitates dopamine-driven light adaptation processes and the retinal circadian regulation. In the absence of melanopsin, the dopaminergic response to the light is limited and light adaptation is incomplete.

The blue light stimulus light 66 used in the method of present disclosure is directed toward the optic nerve head 36 to stimulate the axons of the intrinsically photosensitive ganglion cells (ipRGCs). Based on the physiological aspects described above, the blue light stimulation has been designed to effectively trigger melanopsin expression, which in turn may lead to retinal dopamine release. The increased retinal dopamine is found to have a positive influence on the otherwise further progressing ocular elongation, i.e., axial eye growth, and increasing refractive error in myopic children.

Example

A digital treatment for myopia slowing the progression, and delaying the onset, of myopia is disclosed. In one aspect, the digital treatment enables slowing the progression and/or onset of myopia in children. The method of the digital treatment delivers blue light to the optic disc using a smartphone-compatible game, which is displayed to, and engages the user. The blue light stimulus light 66 is positioned 130 so that it is not visible to the user by directing the blue light stimulus light 66 onto the optic nerve head 36 or optic disc (also sometimes subjectively referred to as the "blind spot"). As noted above, the aim of the blue light stimulus light 66 is to upregulate the retinal dopamine release by activating the intrinsically photosensitive retinal ganglion cells (ipRGCs). The ipRGCs are found in the ganglion cell layer of the retina and contain the photopigment melanopsin, which preferentially absorbs light in the blue range. By targeting the optic disc, the method stimulates the melanopsin in the axons of the ipRGCs. To maximize the dopamine release in the retina, the blue light stimulus light 66 is temporally modulated.

One example of the digital treatment with the method according to the present invention is two short daily sessions that together total less than half an hour.

Stimulus Parameters

The factors influencing dosage have been divided into those attributable to the blue light (stimulus parameters, Table 1) and the impact of the treatment regime (intervention parameters). This section details the relevant characteristics of the blue light stimulus light 66 in an example of the device and the method as tested by the inventors. The importance, selected value for treatment, and rationale for each parameter of the stimulus light 66 are outlined. The intervention parameters are considered in detail in a separate section below.

Table 1. Summary of the stimulus parameters that impact on the dosage of the example of the method of the present disclosure and their associated values. Each parameter is reviewed in detail below.

| Parameter | Value |
| --- | --- |
| Shape | round |
| Size | 4.4 degrees (diameter) |
| Color | RGB 0,0,255 |
| Intensity | 60 melanopic lux |
| Temporal characteristics | 15 Hz |
| Position | over the optic disc |
| Visual content | Diameter of 3.0 degrees |
| Background | black |
| Total stimulus duration per session | 10 minutes |
| Interstimulus interval | approximately 15 seconds |

Shape

For the sake of simplicity and to facilitate overlap with the optic nerve head 36 or optic disc, which tends to be round to oval in shape, the stimulus light 66 is round, for instance substantially circular.

Size

To ensure the correct position 60x, 60y and the size of the stimulus light 66, an understanding of how the size of an object shown, e.g., on the smartphone screen 50, is translated into the size of an image of the object on the retina is helpful. Visual angles are used to indicate the size of the retinal image of the viewed object.

The conversion of linear dimensions (measured, e.g., in mm) into the angles subtended by these linear dimensions on the retina where the image is formed, is referred to as the 'angular formula'.

Deriving the formula

The stimulus light 66, e.g., the blue circle displayed on the smartphone screen 50, passes through two lenses before reaching the retina. A lens in the VR headset and the lens in the eye 30 of the user form a '2-lens system'. The incoming stimulus light 66 is modified by these lenses to form an optical image on the retina of a certain size. Considering the Merge VR headset, which has a lens with focal length of 42mm, the angular formula can be calculated in two steps:

Firstly, the 'magnification factor' (M) is calculated to determine by how much the two lenses magnify the image on the retina, and secondly, the 'visual angle' that the image on the retina subtends is calculated.

Magnification factor M

The focal length of the VR lens is f1 = 42mm. The focal length of the human eye lens is f2=17mm. The distance between the 2 lenses is d = 28mm. The distance between the phone and the VR lens is s01=38 mm.

The formula for obtaining the magnification factor M for a 2-lens system is given by:

$$M = \frac{s_{i1}}{s_{01}} \times \frac{s_{i2}}{s_{02}},$$

where $s_{i1} = \left(\frac{1}{f_1} - \frac{1}{s_{01}}\right)^{-1}, s_{i2} = \left(\frac{1}{f_2} - \frac{1}{s_{02}}\right)^{-1}$ and $s_{02} = d - s_{i1}$.

The magnification was calculated as follows: $s_{i1}$ = (1/42-1/38)-1 = -399 mm; $s_{02}$ = 28 - (-399) = 427 mm; $S_{i2}$ = (1/17-1/427)-1 = 17.704 mm; $M$ = (399*17.704)/(38*427) = 0.435.

The magnification factor M for the Merge VR headset is used to calculate the size of the image on the retina using:

$$size\ at\ the\ retina(mm) = M * size\ on\ the\ smartphone\ screen(mm)$$

This means that anything shown in the smartphone screen 50 through the Merge VR headset is now magnified by a factor of 0.435 on the retina. For example, a dimension of 1 mm on smartphone screen 50 will result in a dimension of 0.435mm on the retina. Visual angle

The visual angle that an object subtends can be obtained using its size in millimeters on the retina. The visual angle is defined as the angle subtended from the center of the human lens (nodal point) to the retina. The human eye 30 is built from the anterior part followed by the thick crystalline lens followed by the vitreous chamber and then the retina. The distance between the center of the lens and the retina is less than the overall axial length.

$$visual\ angle\ \theta°$$

$$= tan^{-1}\left(\frac{size\ at\ the\ the\ retina\ (mm)}{distance\ between\ the\ center\ of\ lens\ and the\ retina\ (mm)}\right)$$

$$= tan^{-1}\left(\frac{size\ at\ the\ retina\ (mm)}{17\ mm}\right)$$

$$= tan^{-1}\left(\frac{M * size\ on\ the\ smartphone\ screen\ (mm)}{17\ mm}\right)$$

$$Visual\ angle\theta° = tan^{-1}\left(\frac{M * size\ on\ the\ smartphone\ screen(mm)}{17\ mm}\right) \qquad \mathbf{2}$$

$$Size\ on\ the\ smartphone\ screen(mm) = \left(\frac{17mm * tan(\theta°)}{M}\right) \qquad \mathbf{3}$$

Applications of the formula

Size of the stimulus: An angle of 4.4° subtended by the diameter of the stimulus light 66, using equation 2, corresponds to diameter of 3.005 mm on the screen 50.

Position 60x, 60y of the stimulus: The location, e.g., angular location, of the optic nerve head 36 (or blind spot) from the fovea 39 is obtained from the fundus image. If the left blind spot had a horizontal angle of 15.5° and a vertical angle of 1.5°, using equation 2, these angular location values of the optic nerve head 36 correspond to a position 60x, 60y on the screen 50 that is displaced by 10.83 mm horizontally and 1.02 mm vertically from the point 55 (fixation point) which corresponds to the location of the fovea 39 on the retina when the user's gaze 33 is directed at the point 55.

Variance of the formula depending on the length of the eyeball

To calculate the visual angles, 17 mm is used as a constant distance between the center of the lens and the retina, and the corresponding focal length of the human eye lens.

The average distance from center of lens to the retina for a child aged 6 to 10 is about 16.2 mm and for a child age 10+ is about 17mm. On the other hand, the length of the eyeball is elongated in myopic children, so longer, rather than shorter, values can be expected.

For a location of the optic nerve head 36 at an angle of 15.5 degrees, the relative difference in the position 60x, 60y of the stimulus light 66 for two different axial eye lengths (i.e., distance from center of lens to retina) of 16.2 mm and 17 mm is about 0.5 degrees. This difference is not significant since there is, as our tests on users have shown, a tolerance range of 0.5 degrees in which the stimulus light 66 remains invisible.

Validation of the Equation

The equation relates the angular size on the retina and the corresponding size on the smartphone screen 50. The equation has been successfully validated in the following ways. Verification using Optics Simulations

Zemax is an industrial grade optical simulation software. The above VR lens-eye system was simulated in the software and the magnification factor, and the visual angles measured in the software were found to be identical to the ones that the formula yields. Success of the fundus calibration

The location, e.g., the angular location, of the user's optic nerve head 36 or blind spot in the retina is obtained, i.e., located 110, using a fundus image obtained using a fundoscopy measurement. The method according to the present disclosure uses the equation (3) to determine, i.e., to position 130, the position 60x, 60y of the stimulus light 66 on the screen 50 which corresponds to the location of the blind spot or optic nerve head 36 on the user's retina. If the result of applying the formula is wrong, then the stimulus light 66 should become visible for the user (since any stimulus light 66 is invisible only at the optic nerve head 36 or the blind spot). A user test (both with informed and uninformed users) applying the formula yielded the desired result, which verified the formula. Hence, the stimulus light 66 was displayed on the screen 50 correctly, i.e., at a position 60x, 60y corresponding to the user's blind spot. See below for more details on a comparison of manual and fundus calibration.

Eye-tracking

'Pupil Invisible' is a wearable eye tracker that tracks the movements of the user's pupil in real time. The wearable eye tracker has an accuracy of about 1 degree. The wearable eye tracker can be worn inside the VR headset.

In a sufficiently large room the user, while wearing the eye tracker, stands facing a wall that is about 1 m away. On the wall, a fixation point is marked, and two stimulus points are provided to either side of the fixation point. The stimulus points were placed at the users' eye level and at a horizontal of 26.3 cm from the fixation point so that the angle subtended by the horizontal distance in the eye 30 is 15 degrees (obtained from equation (3) above where 15 deg. = tan-1 (26.3 cm/1 m)).

While wearing the eye tracker, the user is instructed to first gaze 33 at the fixation point for 10 seconds and then at both the stimulus points for 10 seconds each. The output from the eye tracker gives the value corresponding to an angular value of 15 degrees by taking the difference between the fixation point and the stimulus points

This activity is repeated when the user is wearing the headset and the stimulus light 66 is placed 15 degrees away from a fixation point on the smartphone screen 50 and the user is advised to stare at a stimulus point. If the formula is correct the eye tracker values obtained from the real-world test must be identical to the VR values.

By analyzing the eye-tracking values and fitting it to a normal curve, the following are obtained:

|  | Mean | Std. deviation |
| --- | --- | --- |
| Real world 15 degrees | 0.499 | 0.096 |
| VR 15 degrees | 0.500 | 0.114 |

The formula is considered successfully verified since the eye tracker values are almost identical in both the settings while the standard deviations are significantly larger than difference of the standard deviations.

Validation of the angles being independent of the screen 50.

The stimulus light 66 is displayed by the software app in a way that is independent of the property of the screen 50 (i.e., the resolution and size of the screen 50) and hence should be the same across any smartphone screen 50.

This was verified by measuring the size and distance of the stimulus points from the fixation point for different phone screens 50 using a ruler.

| Phone | Distance between the stimulus points | Size of stimulus light 66 on the screen 50 |
|---|---|---|
| Samsung s7 | 2.1 cm | 0.3 cm |
| Xiaomi Pocophone F1 | 2.1 cm | 0.3 cm |

It was found that the displayed stimulus point was the same size and at the same position 60x, 60y on the screen 50 irrespective of the screen 50 properties for a given VR headset. This meets requirements since the location of the optic nerve head 36 or blind spot in the eye 30 for a user is fixed. For example, if the user's blind spot has a location 15 degrees away from the fovea 39, the stimulus light 66 will be directed at the blind spot (optic nerve head 36) independently of the phone screen 50 properties.

The different VR headsets have different lenses that magnify the phone screen 50 differently. Subsequently, the size of the stimulus point will be adjusted according to the characteristics of the screen 50 to ensure that the user receives the stimulus light 66 having a standardized size and position 60x, 60y.

In conclusion, using different methods it was validated that the visual angle system used in the VR environment corresponds to the visual angle system used in the real world.

The factors that affect the visual angle system in the VR environment are the lens property and the structure of the headset which can be measured and input into the software app for implementing the method. The software app will then ensure matching between any headset and the real-world visual angle system.

Using the visual angle ensures that, e.g., ophthalmological, data from optical systems used in the medical practices, can be directly entered into the software or software app of the present disclosure. For example, an optical system like a fundoscope provides the position and the size of the optic nerve head 36 or the blind spot in angular values. The angular values can be directly entered into the software or software app. The software or software app then directly positions 130 the stimulus light 66 to impinge on the optic nerve head 36 or the blind spot based on the angular values. The properties of the VR headset, for instance of the lenses of the VR headset, affect the visual angles.

To ensure the stimulus light 66 falls within the optic nerve head 36 or the optic disc for all children, a stimulus size of 4.4 degrees visual angle in diameter (2.2 deg. visual angle in radius) is used. This corresponds to 80% of the average optic disc size of the children and thus accounts for natural variations in size across the users. It also enables coverage of the optic cup, the central portion of the optic nerve head 36 or the optic disc, which has a diameter of approximately 2 deg. visual angle in children (mean cup-to-disc size ratio = 0.381-0.386).

A value of the stimulus light 66 that has a size amounting to 80% of the average optic disc size has the effect of reducing the likelihood that the light stimulus falls outside of the optic disc. This size of the stimulus light 66 enables reducing the amount of time of mis-targeted stimulation. To date, both the children and the adults who have tested the method as users have reported that the stimulus light 66 was minimally visible throughout the session. This provides support for the selected light stimulus size.

Intensity

With respect to the method of treating the myopia or the myopia progression as disclosed herein, it is believed that the efficacy of such method is contingent on the activation of the melanopsin in the axons of the ipRGCs at the optic disc. Melanopsin is a photopigment that preferentially absorbs short-wavelength light in the blue range (380-500 nm) of the visible spectrum and is maximally sensitive to light at approximately 480 nm. To stimulate melanopsin at the optic disc, the stimulus light 66 is blue (RGB 0,0,255) and the resultant spectrum of the stimulus light 66 has an intensity of 60 melanopic lux on the screen 50.

Lux is a unit of brightness which is weighted based on the spectral perception of cone cells response (based on the luminous efficiency function). Melanopic lux is a special type of metric according to which the brightness is weighted based on the melanopic cell response instead of the cone cell response. In general, the melanopic lux provides the information on 'the extent to which the melanopsin cell will be activated by the incoming light'. Higher melanopic lux values imply higher melanopsin activation.

The incoming light power spectrum is weighted by its power contribution in terms of $\mu$W/cm2/nm. The contribution of each wavelength bin $\Delta\lambda$ in the spectrum of the incoming light is taken into account.

The incoming power spectrum is weighted based on the melanopsin response curve. The resultant weighted sum gives the melanopic lux value. For example, if the incoming power spectrum has non-zero power only at $\lambda = 640$nm, then the melanopic lux would have a value of zero since red light (approx. 625 nm $\leq \lambda \leq$ 700nm) is 'invisible' for melanopsin. The cones, on other hand, detect red light. Thus, the illuminance of the incoming light having non-zero power at $\lambda = 640$nm only would have > 0 melanopic lux.

The brightness of the blue light stimulus light 66 displayed on the screen 50 of a Samsung Galaxy S7 was measured using i1Studio from X-Rite. The i1Studio from X-Rite provides power spectra (uW/cm2/nm) of the incoming light for every 10 nm from 380 to 730 nm in the form of a csv file.

The i1Studio has two different sensors to measure the ambient and spot brightness. To measure the blue light stimulus light 66, the spot sensor was placed flat against the S7 screen 50 so that the screen 50 faced the blue light stimulus light 66. The brightness was recorded in the 'Spot Measurement Mode'.

The resultant output power spectrum was obtained as a csv file. The file was then imported into and analyzed using the provided online tool (https://fluxometer.com/). This tool calculates the melanopic lux values (CIE S 026/ E 2018 standard) as well as others like the quantal values (in photons/cm2/s) which are useful when comparing with values from literature.

To investigate the consistency of melanopic lux values across different ones of the Galaxy S7 mobile devices, the software or software app of the present disclosure was installed in randomly chosen ones of the Galaxy S7 mobile devices and the resultant brightness of the blue light was measured. The melanopic lux value was on average 58.9 $\pm$2.8, which corresponds to an average of 2.59 $\pm$ 0.11 x 1013 photons/cm2/s.

According to this experiment, the measured melanopic lux value of 58.9 $\pm$ 2.8 is the value that will be taken as a baseline for the stimulus light 66 in the clinical study. The experiment verified that melanopic lux values are consistent across the different Galaxy S7 mobile devices. In the method of the present disclosure, melanopic lux is the unit used to compare stimulus light 66 from different displays 50 or screens 50 (be it from the different Galaxy S7s or from other mobile devices).

Based on the experiment, a Galaxy S7 mobile device can be used in the clinical trial, without the need to measure or calibrate the individual ones of the Galaxy S7 mobile device individually. Rough visual inspection by eye care professionals may take place, and any perceived abnormalities will lead to further inspection of the Galaxy S7 mobile device. In case of the organic light emitting diodes (OLED) displays or screens 50, the red and green OLED films have lifetimes of 46,000 to 230,000 hours and blue organics currently have lifetimes of around 14,000 hours. For an average yearly screen time of 1,500 hours, no display degradation (screen degradation) is expected that affects the brightness of the stimulus light 66.

The brightness of the stimulus light 66 reaching the optic nerve head 36, or the optic disc, depends on multiple parameters, including the focal length of both the VR lens and the eye lens, transmission of the lenses, spectrum filtering and scattering of the lenses, shape of the lens, distance between the screen 50, e.g., a smartphone or mobile device, and the eye 30, among others.

The primary contribution from these factors is the light transmission of both the VR lens and the eye lens which can be readily measured. The contributions from other factors like the scattering, the distance between the mobile device and the eye 30, a distance between VR lens and the eye lens are all negligibly small in comparison even across different headsets.

Since only one type of mobile device and one VR headset were used throughout the study, the mentioned factors had no effect (for example, the transmission was always the same due to using the same headset) and the brightness at the mobile device surface alone provides sufficient information.

In case of using other mobile devices, the methodological approach described above is applicable. More parameters, such as display technology and brightness, screen resolution and spectral output, display size and curvature, and software compliance, need to be considered when using the other types of headsets. The size and the position 60x, 60y of the stimulus light 66 on the screen 50 may change and the number of photons arriving at the eye 30 will have to measured. The effect of other factors is measured if they are found to have an impact on the brightness output.

Melanopic lux was chosen as the unit of measurement, as the melanopic lux reflects the radiance of light weighted according to the spectral sensitivity function of melanopsin. In doing so, the measurement of melanopic lux incorporates both the brightness and the spectral composition of the light source 60 and provides a value that is indicative of the intensity of light that affects the melanopsin. Assuming all other parameters are unchanged, the melanopic lux is the unit that determines the impact of the method according to the present disclosure. The value of the melanopic lux is also dependent on the size of the light source 60. A value of 60 melanopic lux corresponds to a blue light stimulus light circle of a radius of 2.2 deg. on the smartphone screen 50.

It was demonstrated that melanopic lux from the light source 60 is sufficient to activate the melanopsin by assessing the pupil light response to blue light stimulation of the optic disc (Schilling et al., 2020).

To determine whether melanopic lux stimulates the retinal dopamine via the ipRGC activation in humans, contrast sensitivity was measured as an indirect measure of the dopamine release. Administration of levodopa and nomifensine, both of which are dopamine agonists, to healthy adults was previously shown to improve the contrast sensitivity. Similarly, a significant improvement in medium to high spatial frequency contrast sensitivity was measured after blue light stimulation of the optic nerve head 36 or optic disc. Therefore, the results of this study provide evidence that the blue light stimulus light 66 can modulate retinal processes that are regulated by the retinal dopaminergic system.

Temporal characteristics

The blue light stimulus light 66 is temporally modulated with, e.g., a rectangular waveform and, e.g., a frequency of 15 Hz. Research in several animal species has revealed that flickering light stimulates the dopamine release and can be more effective at doing so than steady light. In general, low frequency (< 4 Hz) and higher frequency (20 Hz) flicker may reduce dopamine synthesis in the retina and can induce a myopic shift. On the other hand, moderate flicker frequencies (approximately 6-15 Hz) have been found to suppress experimentally induced myopia and increase retinal dopamine synthesis. Thus, an intermediate frequency of 15 Hz should complement the dopamine-stimulating effect of the blue light. A 15 Hz flickering blue light stimulus light 66 has been used successfully in the experiments in humans.

Position

For the blue light stimulus light 66 to activate the melanopsin in the axons of the ipRGCs, the blue light stimulus light 66 is positioned 130 such that the blue light stimulus light 66 impinges on the optic disc of each user. This is achieved by determining, i.e., locating 110, the location, e.g., the angular location, of the child's optic nerve head 36 or optic disc via fundoscopic, i.e., ophthalmoscopic, imaging performed, e.g., by an ophthalmologist. The location of the optic disc is obtained, i.e., located 110, in the horizontal and vertical direction with respect to the fovea 39 (fixation). The location of the optic disc is provided in either degrees or micrometers depending on the software. In the case that the coordinates are given in micrometers, the values in degrees can be obtained by a simple calculation. This information is entered into the device 10 and/or the software app executed on the processor 80, which then positions 130 the stimulus light 66 according to the unique physiology of each child.

'Calibration' here refers to the process of positioning 130 the blue light stimulus light 66 with the radius of 2.2 degrees on the optic nerve head 36, or the optic disc, of the user's left and right eyes 30, e.g., on the center of the optic nerve head 36. Positioning 130 of the blue light stimulus light 66 such that the blue light stimulus light 66 impinges on the optic nerve head 36 (or the optic disc / blind spot) is to be understood to have occurred, i.e., the device 10 (e.g., the display/screen 50 or the VR headset) has been calibrated, when there is an overlap of the stimulus light 66 and the optic nerve head 36 at the retina.

Manual calibration:

During manual calibration, the user fixates on the fixation cross and uses the controller, e.g., a Bluetooth controller, to move, i.e., to adjust the position 66x, 66y, the blue light stimulus light 66 within the screen 50 so that the blue light stimulus light 66 perceptually falls inside, or overlaps with, the optic nerve head 36 or the blind spot when fixating on the fixation cross. Overlap at the retina of the optic nerve head 36 or the blind spot, e.g., the center of the optic nerve head 36, and the position 60x, 60y of the blue light stimulus light 66 is perceptually identified when the blue light stimulus light 66 is 'invisible'. Fundus calibration:

Fundus imaging provides a picture of the retina including the fovea 39 and the optic nerve head 36. The fundus imaging enables determining the distance, e.g., angular distance, between the fovea 39 and the optic nerve head 36. In some fundoscopes, angular values are directly output. The angular values may also be manually obtained by measuring the distances using the fundus picture and a ruler. These angular values can be entered into the software app and/or the device 10, which positions the blue light stimulus light 66 accordingly by means of the processor 80, which communicates with the light emitting source 60.

Method

The fundus image is obtained without any cycloplegia. In the fundus image, the optic nerve head 36, e.g., the center of the optic nerve head 36, (and hence the blind spot) is identified as the part where the central retinal blood vessel is located. The distance, e.g., angular distance, between the fovea 39 and the optic nerve head 36 can be obtained using 2 methods:

Method 1:

By measuring the distance in millimeters (mm) between the fovea 39 and the optic nerve head 36 and converting the distance to angular values as follows:

identify the field of view (in degrees) of the fundoscope (this is typically 30 degrees or 45 degrees.

Print out the fundus image and using the ruler to measure the distance (in millimeters) between the edges of the image. This would correspond to the field of view of the fundoscope. Then obtain a ratio by dividing the fundoscope angle by the paper width. Finally measure the distance between the fovea 39 and the optic nerve head 36 or blind spot. Use the ratio to obtain the angular distance between fovea and the optic nerve head 36

Method 2:

By obtaining the angular values directly from the fundus imaging software

Results

| Subjects | Age | Manual calibration (angles) | Fundus calibration (angles) |
|---|---|---|---|
| Subject1 | 12 | <table><tr><td>Left X</td><td>Left Y</td><td>Right X</td><td>Right Y</td></tr><tr><td>16.9</td><td>1.4</td><td>17.4</td><td>1.9</td></tr></table> | <table><tr><td>Left X</td><td>Left Y</td><td>Right X</td><td>Right Y</td></tr><tr><td>15.52</td><td>0.523</td><td>15.17</td><td>-0.34</td></tr></table><br>*The user found this fundus calibration objectively invisible |
| Subject2 | 10 | <table><tr><td>Left X</td><td>Left Y</td><td>Right X</td><td>Right Y</td></tr><tr><td>14.8</td><td>1.2</td><td>15.4</td><td>2.3</td></tr></table> | <table><tr><td>Left X</td><td>Left Y</td><td>Right X</td><td>Right Y</td></tr><tr><td>14.2</td><td>0</td><td>17.23</td><td>1.531</td></tr></table> |
| Subject3 | 18+ | <table><tr><td>Left X</td><td>Left Y</td><td>Right X</td><td>Right Y</td></tr><tr><td>15.6</td><td>1.5</td><td>15</td><td>1.5</td></tr></table><br><table><tr><td>Left X</td><td>Left Y</td><td>Right X</td><td>Right Y</td></tr><tr><td>15.58</td><td>2.541</td><td>15.415</td><td>1.524</td></tr></table> | <table><tr><td>Left X</td><td>Left Y</td><td>Right X</td><td>Right Y</td></tr><tr><td>15</td><td>2.7</td><td>14.8</td><td>1.744</td></tr></table> |
| Subject4 | 18+ | <table><tr><td>Left X</td><td>Left Y</td><td>Right X</td><td>Right Y</td></tr><tr><td>15.5</td><td>0.4</td><td>15.7</td><td>1.5</td></tr></table><br><table><tr><td>Left X</td><td>Left Y</td><td>Right X</td><td>Right Y</td></tr><tr><td>15.5</td><td>0.2</td><td>15.6</td><td>1.5</td></tr></table> | <table><tr><td>Left X</td><td>Left Y</td><td>Right X</td><td>Right Y</td></tr><tr><td>15.81</td><td>0.27</td><td>15.81</td><td>1.36</td></tr></table> |

Additional users were tested using fundus calibration only.

| Subject 5: | <18 | NA | |
|---|---|---|---|

| Left X | Left Y | Right X | Right Y |
|---|---|---|---|
| 13.95 | 4.27 | 13.67 | 0.28 |

| Subject 6: | 7 | NA | |
|---|---|---|---|

| Left X | Left Y | Right X | Right Y |
|---|---|---|---|
| 15.81 | 0.27 | 15.81 | 1.36 |

The fundus calibration method is successfully verified to position 130 the stimulus light 66 on the screen 50 such that the blue light stimulus light 66 impinges inside the optic nerve head 36, which leads to better invisibility of the blue light stimulus light 66:

The users were asked to provide their feedback on the fundus calibration method immediately after conducting manual calibration. The users (two children aged in the range 7-11 and two adults aged 18+), with prior experience with the manual calibration, found that the fundus calibration leads to better invisibility.

The users also used the calibration values for multiple sessions and found that the blue light stimulus light 66 was invisible in all sessions.

Additional children were tested on the invisibility of the fundus calibration but without direct comparison to the manual calibration. Three additional children aged 6 to 14 were provided with the fundus calibration and feedback was obtained on the invisibility of the stimulus light 66. All three children found the blue light stimulus light 66 to be invisible.

Fundus calibration is more reliable than manual calibration:

Over repeated manual calibrations, higher variance is found in manual calibration in comparison to the fundus calibration due to its subjective nature. Since the area of the optic nerve head 36 is larger than the area of the retina the blue light stimulus light 66 impinges on, there is more freedom for the user to place the blue light stimulus light 66 inside the optic nerve head's area, which might not be exactly at the center. Positioning 130 the blue light stimulus light 66 such that the blue light stimulus light 66 impinges at the center of the optic nerve head 36 is preferred. Such positioning 130 reduces the visibility of the blue light stimulus light 66 in case of micro eye movements in any direction.

Due to the objective nature, the variance in the fundus calibration is minimal in comparison to the manual calibration. Fundus calibration enables positioning 130 the blue light stimulus light 66 such that the blue light stimulus light 66 impinges at the center of the optic nerve head 36. Such positioning 130 enables equal blind spot invisibility in all possible directions of eye movements and thus decreases the probability of the blue light stimulus light 66 becoming visible.

The fundus calibration provides better tolerance than manual calibration:

After testing the fundus calibration, the fundus values were changed +/- 0.5 degree and +/- 1 degree to test the limit before which the blue light stimulus light 66 becomes visible. The fundus calibration had a tolerance of 0.5 degrees in the horizontal direction before the blue light stimulus light 66 was visible again.

Younger children do not find the manual calibration user-friendly:

From manual tests, the users, especially younger children, find that the manual calibration is difficult to perform. Manual calibration involves some understanding of when the blue light stimulus light 66 becomes perceptually invisible and of how to recognize invisibility. For younger children aged 6 to 8, it is not intuitive to stare at the fixation point for longer durations and move the blue light stimulus light 66 using various button combinations and at the same time to perceptually identify the optic nerve head 36 or the blind spot. Image based calibration provides freedom for the user to skip these steps and hence it presents a more user-friendly approach.

After the blue light stimulus light 66 is positioned 130 by the software app, the user focuses the gaze 33 within the target area 52 of the screen 50 to maintain the blue light stimulus light 66 on the optic disc. Care has been taken to tailor the content provided by the software app, e.g., a virtual reality (VR) game, to facilitate fixation of the user's gaze 33, in particular relative to the visual content presented to the user and the background of the screen 50.

In a further aspect of the invention, the device 10 provides automated calibration. In this aspect, the optic nerve head 36 is located based on one or more statistical parameters. The statistical parameters may be based on a set of measured locations of the optic nerve head 36 for a group of users. The measured locations of the optic nerve head 36 may originate, e.g., from health records or data collected during manual calibration of the device 10. The group of users may have specified features such as, e.g., age. The statistical parameters comprise mean and standard deviation but are not limited thereto.

The location of the optic nerve head 36 may be determined simply on the mean of the location of the optic nerve head 36 for the group of users. The mean has, for example, a value of approximately 15 to 16 degrees.

**In** another aspect, the device 10 may request feedback from the user and adjust the location of the optic nerve head 36 based on the feedback and, e.g., the standard deviation of the location of the optic nerve head 36 for the group of persons.

Visual content

The visual content provided by the software, or the software app is limited to a target area 52 or "focus circle" of the screen 50, corresponding to an area in the foveal region 39 of the user's eye 30 having a diameter of approximately 3.0 degrees. The area in the foveal region 39 of the user's eye 30 may be a circularly shaped area in the central foveal region 39 having a radius of approximately 1.5 degrees. By presenting the content, e.g., salient game content, within the target area 52 of the screen 50, a user's gaze 33 is maintained within the target area 52 of the screen 50 to facilitate continuous optic disc stimulation. The size of the focus circle, i.e., the target area 52 on the screen 50, is calculated using eye tracking data. More details are provided below.

While displaying the content provided by the software app, e.g., the game the user engages with, the screen 50 is set to full brightness to ensure that the blue light stimulus light 66 has a brightness of ~60 melanopic lux. **In** the otherwise dark environment of the VR headset, this brightness causes the content provided by the software app to appear very bright and highly contrasted for close viewing. Hence, the content provided by the software app contrast is decreased to ensure better usability, reduce eye strain and to minimally affect the treatment light cascade.

An alpha channel filter was implemented on top of the content provided by the software app so that light from the content provided by the software app is 'dimmed' before reaching the user's eye 30. The resulting contrast is therefore reduced to balance all the above-mentioned parameters.

Measuring the brightness of full white without a filter: To test the maximum possible brightness that the content provided by the software app delivers without any alpha channel, the target area 52 of the screen 50 displayed full white light and the resultant brightness was measured while using the treatment. The brightness was measured to be around 130 melanopic lux without the alpha channel.

Measuring the maximum brightness of full white light but through an alpha channel filter to reduce the brightness: To ensure better viewing comfort, reduce eye strain and minimize the influence on the treatment cascade, an alpha channel filter was introduced to cover the target area 52 so that the contrast of light from the content provided by the software app is minimized. The alpha channel filters take values from 0 to 1 where 0 does not allow any light to pass through and 1 allows all the light to pass through. The resultant alpha channel value was chosen to be 0.7.

To test the maximum possible brightness that the content provided by the software app delivers through this alpha channel, the target area 52 on the screen 50 displayed full white light and the resultant brightness was measured. The brightness was measured to be around 30 melanopic lux which is significantly reduced compared to using no filter. From user tests, the reduced brightness based on the alpha channel filter proved to be more comfortable for the users.

Measuring the average brightness of the game icons: The full white icon is an ideal situation for measuring the maximum brightness since white has contribution from the entire visible spectrum. The icons used while playing the game have colors and therefore have a modified spectrum in comparison to the full white color. The modified spectrum leads to less brightness in comparison to the white light spectrum.

To accommodate for the substantial number of icons that are used, the average brightness was measured by choosing randomized icons from different icon sets and measuring their brightness. The average brightness was measured to be around 11 melanopic lux which is significantly less than the full spectrum of white light brightness.

Conclusion

To ensure better usability, reduce eye strain and to minimally affect the treatment light cascade, the light from the content provided by the software app was reduced by adding an alpha channel filter with a value of 0.7. The average brightness of the icons used in the content provided by the software app was found to be around 11 melanopic lux which is a reduction of more than 90% in melanopic lux. This is significantly less bright compared to the condition when no filter is used. Since this setting was proven to be well-perceived during the user testing, this value is used in the investigational device.

Any visual content presented to the user that meets the same requirements for fixing the user's gaze 33 inside the focus circle, i.e., the target area 52 of the screen 50, will be considered acceptable. **In** the case that the presented visual content does not support a similar degree of fixation of the user's gaze 33 within the focus circle (i.e., approximately 60% - see below), additional analyses will be performed to determine if the stimulation duration should be adapted to achieve an equivalent effective stimulation duration.

Background of the screen 50

The area outside of the focus circle or target area 52 of the screen 50, termed the background of the screen 50, is dark, e.g., black. The black screen background encourages users to maintain their gaze 33 within the focus circle or target area 52 of the screen 50. The dark screen background further enables controlling an effect the visual content, other than the blue light stimulus light 66, has on the treatment. Furthermore, the dark screen background enables dim light adaptation of the user's eye 30, which increases the ipRGC sensitivity to the blue light stimulus light 66 and thus evokes a larger response. The dark screen background also allows for a lower radiance of the blue light stimulus light 66 to be used compared to a bright one of the background, thereby ensuring user safety and comfort. When the screen 50 is combined with, or inserted into, the VR headset, any light apart from the stimulus light 66 and the light representing the content, e.g., ambient light or light from outside the position 60x, 60y or the target area 52, is thus blocked from reaching the eyes 30 of the user.

Despite these efforts to maintain the blue light stimulus light 66 over the optic disc or the optic nerve head 36, the eye movements may result in a portion of the stimulus light 66 being off-target and not reaching the optic disc or the optic nerve head 36 for part of the session. To ensure melanopsin activation, and thus treatment efficacy and usability, only a crescent section amounting to 30% of the round, e.g., substantially circular, shape of the stimulus light 66 is tolerated to fall outside the optic nerve head 36 or the optic disc.

Eye tracking data acquired from a group of the users, i.e., six adults and two children, determined that with a salient focus circle or target area 52, users naturally abide by this "30% rule" relatively well. The obtained fixations of the users' gazes 33 formed a Gaussian distribution around the visual content and revealed that, on average, users stay within the allowable range approximately 60% of the time. Therefore, when the eye movements are taken into consideration, the blue light stimulus light 66 is on average positioned 130 such that the blue light stimulus light 66 impinges on or within the optic nerve head 36 or the optic disc for approximately 60% of the time. This duration is referred to as the effective stimulation duration and is, in essence, the amount of time the user actively receives the treatment. These factors were considered when specifying the stimulus duration, as detailed below.

Performance of fixation of the user's gaze 33 was also considered when determining the size of the focus circle (or the target area 52). With a maximum visible crescent section, amounting to 30% of the round shape of the stimulus light 66, and an average fixation performance of 60%, the focus circle corresponding to an area in the foveal region 39, e.g., the central foveal region 39, having a diameter of approximately 3.0 deg., e.g., a radius of approximately 1.5 deg., adequately limited eye movements outside of the focus circle (i.e., target area 52). When the user sufficiently adheres to the focus circle (i.e., fixates the gaze 33 within the target area 52), the stimulus light 66 is maintained on the optic nerve head 36 or the optic disc for a significant portion of the session. More details are provided below.

Stimulus duration

In contrast to the theoretical concept of effective stimulation duration (defined above), stimulus duration refers to the total amount of time the flickering blue light stimulus light 66 of the method according to the present disclosure is present on the smartphone screen 50. Assuming stable fixation, the stimulus duration enabling blue light stimulation of the optic disc and upregulation of retinal dopamine release is approximately 60 seconds. Based on the findings from electroretinogram (ERG) research, 60 seconds of stimulation is sufficient to induce a significant increase in the retinal electrical activity of myopic individuals that likely involves the dopaminergic system. This effect is greatest 20 minutes after stimulation but continues to be observed 60 minutes after stimulation. Given the estimated fixation performance when implementing the method according to the present disclosure (see section "Position"), to ensure that 60 seconds of effective stimulation is achieved, the treatment must be used for at least 100 seconds (stimulus duration) to have the same effect as demonstrated in the experiment above.

While a duration of 60 seconds of stimulation is sufficient to activate the retinal dopamine, analyses have revealed that stimulation for 10 minutes may support a greater effect. After 10 minutes of blue light stimulation of the optic disc, the retinal electrical response is elevated 60 minutes following removal of the stimulus. A sustained response is seen as being more favorable to induce the dopamine-initiated signaling cascade to slow the ocular growth. To facilitate treatment usability and adherence, a stimulus duration of 10 minutes is recommended, corresponding to an effective stimulation duration of approximately 6 minutes per session. This effective stimulation duration assumes an average fixation of 60%. However, somewhat lower fixation performance is not expected to have a meaningful impact on treatment efficacy (see FIG. 3). Interpolation between 10 seconds (no change) and 60 seconds (significant change) of the stimulation suggests that at least 30 seconds of stimulation should be sufficient to achieve an effect. Peak ipRGC firing should be achieved after approximately 30 seconds of stimulation. Therefore, a 10-minute stimulus duration when implementing the method of the present disclosure may be the sum of several shorter presentations of the blue light stimulus light 66 each with a minimum duration of around 30 seconds. In one aspect of the disclosure, the stimulus duration is at least one minute. In a further aspect of the present invention, the stimulus duration is no more than 20 minutes.

Interstimulus interval

In an example of the device and the method according to the disclosure as tested by the inventors, the gamified content that facilitates delivery of the blue light to the optic disc was divided into a variety of "levels". Between these levels, blue light stimulation does not take place. These short breaks of at least 15 seconds are referred to as interstimulus intervals. These breaks allow the child to blink and look around freely inside of the virtual reality headset. During the interstimulus intervals, children are presented with an exit screen indicating termination of the previous level followed by an introductory screen for the upcoming level. The aim of these breaks in treatment is to minimize ocular strain and any associated effects, such as dry eye, while keeping the user engaged in the gameplay and in a state of dim light adaptation. As the blue light stimulus light 66 is not presented during the interstimulus interval, the total duration of the breaks is not included in the stimulus duration, but rather in the overall session duration.

These breaks, or interstimulus intervals, may also support the efficacy of the treatment. While the ipRGCs are capable of continuously responding to prolonged periods of exposure to ambient light, like traditional photoreceptors, the ipRGCs are desensitized following light exposure. This means that with continuous light exposure, the ipRGCs become less responsive to light. Given the intensity of the stimulus light 66 according to the present disclosure, maximum responsivity will be achieved within the first 60 seconds of exposure, after which the ipRGCs will slowly repolarize. The idea is that by returning the ipRGCs to relative darkness (i.e., no blue light stimulation) during the interstimulus intervals, they will begin to return to their baseline state and be able to respond more strongly when the blue light stimulation resumes.

Intervention Parameters

The details of the blue light stimulus light 66 parameters and the impact of the parameters on the treatment with the method according to the present disclosure have been provided above. In addition to the parameters of the stimulus light 66 itself, temporal aspects of implementing the method also influence the outcome. Information about these parameters and useful exemplary values (Table 2) are provided in the following table 2.

Table 2. Summary of the intervention parameters that impact the dosage of the method according to the disclosure and their associated values. Each parameter is reviewed in detail below.

| Parameter | Value |
| --- | --- |
| Session duration | approximately 12-15 minutes |
| Time of use | morning (before 13:00), midday (after 11:00) |
| Frequency of use | twice per day |
| Intersession interval | at least 2 hours |
| Length of treatment | approximately 2 years, for the course of progression |

Session duration

The session duration refers to the total amount of time required to perform the method according to the present exemplary setup. The session duration includes the stimulus duration, the sum of breaks between levels, and the amount of time required to set up and end a session (e.g., arranging the VR headset, turning on and off the device 10). Depending on the age and technological literacy of the child, the session duration will not be longer than 15 minutes. In another aspect of the disclosure, the session duration will be at least 1 minute. In yet another aspect of the disclosure, the session duration will be no more than 30 minutes. Preferably, the session duration will be in the range of 12 to 15 minutes.

Frequency of use

**In** this exemplary setup, the treatment schedule consists of two daily sessions. Implementing multiple sessions per day supports both the efficacy and usability of the method according to the present disclosure. Although the retina-to-sclera signaling cascade remains unknown, it is likely that the regular and/or sustained dopamine release generates the dopaminergic signal to alter the ocular growth and engage subsequent mechanisms. By having two sessions each day, a reinforced dopamine release as well as a sustained dopamine response are enabled. For instance, the two sessions may be timed such that from a first morning session, when the treatment is expected to be most effective, to a second midday session, when the choroid is thinnest, the dopamine release and the dopamine response are elicited.

Time of use

The treatment according to the method of the present disclosure is preferably performed during daytime. **In** one aspect, it is recommended that, in the case of a child, the first session take place in the morning before school followed by a second session immediately after school. The second session should be performed as close to midday as possible. To ensure that the treatment is performed both in the morning and in the afternoon the following time windows may be used: 7:00-13:00 and 11:00-18:00. One session may be completed in each of the time windows while adhering to the intersession interval defined below. The overlap of the two windows between 11:00 and 13:00 ensures the child has the opportunity to perform a session at noon; however, only one session is preferably performed between 11:00 and 13:00 in accordance with the intersession interval. The later session is preferably completed at least three hours prior to the child's normal bedtime to minimize any potential effects of the blue light stimulus light 66 on the circadian rhythm.

The morning session enables temporal overlap with a peak in melanopsin protein expression, which generally occurs at dawn. A session in the morning thus enables treatment according to the method of the present disclosure when melanopsin expression is high and ipRGCs are likely to respond more efficiently to the blue light stimulus light 66. As a result of high melanopsin expression, retrograde signaling to dopaminergic amacrine cells is supported.

The midday session enables making use of diurnal rhythms in the eye 30. The diurnal rhythms enable optimizing treatment efficacy. **In** humans, the choroid is thinnest in the early afternoon, at approximately the same time that the axial length of the eye 30 is longest. There is evidence suggesting that choroidal thickness changes provide a short-term biomarker of vision-dependent mechanisms that regulate eye growth and precede longer-term changes in eye size. Visual stimuli with known anti-myopiagenic effects and processes leading to emmetropia and hyperopia are associated with choroidal thickening. On the other hand, processes leading to myopia are accompanied by choroidal thinning. A session of the method of the present disclosure at midday enables choroidal thickening when the choroid is typically thinnest and providing a signal to inhibit ocular growth.

Intersession interval

Between the two daily sessions, an inter-session interval of at least two hours is preferably observed. The inter-session interval of this length should reinforce dopamine release across the two sessions and encourage the users to perform the treatment in both the morning and at midday.

Investigation of the time course of the retinal electrical response to the blue light stimulation of the optic disc revealed an effect 60 minutes after the stimulation with the blue light of a duration of 10 minutes. An increase in the retinal electrical response was also observed 60 minutes after the blue light stimulation of a duration of 60 s, however, to a lesser degree. Thus, a retinal response to the blue light stimulus light 66 continues to be measurable at least 60 minutes after the stimulus is removed. It is assumed that the influence of the blue light stimulus light 66 will begin to degrade and return to baseline at some time after the 60-minute mark. This is consistent with the ipRGC response, which has been found to persist for at least one hour after light-off following prolonged exposure to a slightly dimmer stimulus light 66. Therefore, it is suggested that the second session be performed no earlier than two hours after completion of the first session.

Duration of treatment

The total treatment duration may be, for example, two years. However, the duration of treatment is not limited to this length. The treatment with the method according to the present disclosure is considered to be clinically meaningful as long as myopia progression is detectable. As noted above, ideally, the children would use the method twice a day during the recommended treatment windows. In other aspects, the method may be performed three times a day or up to five times a day. A single daily session is considered successful if at least 80% of the treatment duration is completed and the user actively played, i.e., was engaged with, the VR game (which is tracked via log data). At least 75% of the total sessions over the course of two years will need to be completed with no interruption longer than four consecutive weeks for the entire treatment to be considered successful (i.e., "per protocol").

The treatment according to the method of to the present disclosure is based on a number of stimulus and intervention parameters. The stimulus and intervention parameters include the characteristics of the blue light stimulus light 66, as well as factors associated with using the treatment. Together, these parameters influence the efficacy and usability of the method according to the present disclosure. The inventors have found stimulus and intervention parameters that provide an effective treatment that are easy to implement. The stimulus and intervention parameters result in an upregulated retinal dopaminergic system by stimulating the optic nerve head 36 or the optic disc with blue light, e.g., emitted 170 from the Samsung Galaxy S7. At 60 melanopic lux, the blue light stimulus light 66 is sufficient to activate the melanopsin-containing ipRGC axons and retrogradely induce the release of the dopamine from the amacrine cells in the retina. The blue light stimulus light 66 is delivered, i.e., emitted 170, via or alongside an entertaining game application and positioned 130 over or overlapping with a portion of the user's visual field 37, e.g., in the target area 52 of the screen 50, corresponding to the optic disc. **In** other words, the blue light stimulus light 66 is positioned 130 in the user's visual field 37 such that the blue light stimulus light 66 impinges on the optic nerve head 36 of the user. The game is divided into short levels to maximize the response of the ipRGCs and the user engagement. By using a salient and centrally focused game, effective stimulation of the optic nerve head 36 or the optic disc can be ensured throughout the treatment sessions. The method according to the present disclosure may be used twice a day, i.e., with two treatment sessions per day, each lasting for about 12 minutes (i.e., a session duration, which includes setup, stimulation with a 10-minute stimulus duration, breaks between levels, and termination), ideally in the morning and at midday, to take advantage of existing diurnal ocular rhythms.

An aim was to measure the fraction of duration that the users receive the round, e.g., circular, blue light stimulus light inside their optic nerve head 36 or the blind spot (hence non-image forming and perceptually invisible stimulus) during the gameplay duration. A stimulus is defined to be outside the blind spot (perceptually visible) when more than 30% of the stimulus radius is outside the blind spot. A total of 8 users or participants (6 adults, 2 children) were recorded.

Selected users focus the gaze 33 at the fixation cross and at 3 degrees to the left, right, top, and bottom of the fixation cross. Focusing the gaze 33 at pre-defined positions with respect to the fixation cross enables calibrating the eye-tracking. This calibration enables matching the value of each degree of the visual field 37 to the values output by the Pupil Invisible eye tracker, an eye tracker provided Pupil Labs in the form of spectacles or goggles that can be worn inside VR goggles. The Pupil Invisible eye tracker samples and/or records data at a frequency of 200 Hz and with a resolution of 1 to 2 degrees.

The users were asked to play the reaction game for 2 to 6 minutes and the eye movements are recorded using the Pupil Invisible eye tracker. The game was displayed inside the target area 52 of the screen 50 corresponding to an area having a diameter of 3.0 degrees, e.g., a circularly shaped area having a radius of 1.5 degrees, in the foveal region 39, e.g., the central foveal region 39.

The fixations of the users' gazes 33 and the eye movements were extracted from the Pupil Invisible's 'pupil player' software. The fraction of duration during which the users received the round blue light stimulus light 66 at the optic nerve head 36 for different durations of the game was then analyzed from the data. A constraint was that the users were to visibly perceive maximally 30% of the radius of the stimulus.

The measurements were repeated and compared for different content apps, i.e., for different games, displayed to the user.

For each measurement, the dosage parameters were identified, e.g., the size of the stimulus, maximal blind spot stimulus visibility during gameplay, and duration of the treatment were recorded.

Method

As noted above, eight users (participants) (6 adults, 2 children) participated in the trial. The selected users calibrated the eye-tracking by looking at the fixation cross and to 3 degrees to the left, right, top, and bottom of the fixation, which enables matching the value of each degree of the visual field 37 to the values output by the Pupil Invisible eye tracker.

The users were asked to play the reaction game for 2 to 6 minutes and the eye movements were recorded. The content, i.e., the game, was displayed inside the target area 52 corresponding to an area in the foveal region 39, e.g., the central foveal region 39, with a diameter of 3.0 degrees, e.g., a radius of 1.5 degrees.

The recordings automatically uploaded to the Pupil Cloud and were downloaded as raw data. The Pupil Lab outputs a value for (x,y) between 0 to 1 where 0.5 is the center.

The software that comes along with Pupil Labs called Pupil Play v2.4.0 automatically analyses the raw data to extract the fixations in an excel .csv file. The parameters to extract the fixation from the software were: Dispersion: 1.2; Minimum fixation duration: 100ms; Maximum fixation duration: 450ms.

Choosing the key performance indicator (KPI) for gaze stability and method of analysis
The content provided by the software or software app, e.g., a reaction game, is designed in such a way that the user has to focus the gaze 33 on a very small target area 52 of the screen 50 (<<1.5 deg.) to play the game successfully. The fixations of the users are distributed according to a normal distribution around the 'area of interest' (target area 52) on which the user has to focus the gaze 33. Depending on how well the user performs, the normal distribution might be very narrow (if the user performs very well, they move the gaze 33 away very little) and if the user performs badly then the normal distribution is very wide (the user moves the gaze 33 everywhere but predominantly to the 'area of interest'). The extracted normal distribution gives two values 'mu' and 'sigma' where 'mu' is the center of the distribution and 'sigma' is the standard deviation of the distribution.

The assumption that the users must see a portion of the stimulus light 66 corresponding to 30% of the stimulus radius (2.2 deg.) before the stimulus light 66 becomes "visible" results in an allowable maximum range of eye movement of 1.21 degrees from the focus of the gaze 33 (i.e., the fixation cross). Fixation of the gaze 33 outside this range may result in the stimulus light 66 becoming visible and may reduce stimulation of the optic disc, or optic nerve head 36. The user does not fixate the gaze 33 if the eye movements exceed the maximum allowable range of 1.21 degrees from the fixation cross or center of the target area 52 (focus circle) (FIG. 17). The maximal allowable eye movement can be calculated as (size of blind spot - the size of stimulus) + 30% of the size of stimulus).

Thus, the normal distribution of the focus of the gaze 33 was plotted and the percentage of values lying between the allowable eye movements gives the 'fixation performance'.

This **KPI** was chosen because the KPI is very scalable and sufficiently accurate performance results are swiftly obtained. Other KPIs that involves time stamps are not very accurate and not scalable.

Results

Calibration finding

From the calibration analysis, the 1.21 degrees of visual angle for the allowable eye movement subtended a value of 0.0123 from the eye tracker values which was consistent across users. This means that each degree of visual angle corresponds to the same eye tracker values for all users.

This means that it is not necessary to calibrate for every subject. It is sufficient to record the gameplay and look at the values that lie between 0.0123 eye tracker range of the normal distribution obtained from the fixation data.

Performance calculation result

The size of the blue light stimulus light was set to have a radius of 2.2 degree and the maximally allowable eye movement was set to 30% of the radius of stimulus size.

| Subject | Fixation performance |
| --- | --- |
| Subject1 | 69.5299 |
| Subject2 | 42.592 |
| Subject3 | 59.311 |
| Subject4 | 67.8285 |
| Subject5 | 56.7543 |
| Subject6 | 76.6483 |
| Subject7 | 37.0394 |
| Subject8 | 56.7543 |

The probability distribution of values lying between the allowable eye movements (= 0.0123 range) gives the 'fixation performance' (see table above). From analyzing 8 users (6 adults, 2 children), the performance was on average 60%.

Dosage parameter identification

There are two parameters that affect performance: the size of the stimulus and the maximally allowed deviation of the stimulus light 66 from the optic nerve head 36. The radius of the stimulus was set to 2.2 degree and the maximally allowable deviation of the stimulus light 66 from the optic nerve head 36 to 30% of the radius of the stimulus.

From analyzing the eight users, the average performance was determined to be 60%. One of the child users had a better-than-average performance of 76%.

At least 1 minute of constant stimulation of the optic nerve head 36 or blind spot to obtain the medical effect in combination with 60% fixation performance of users yields a minimum stimulation duration of 1.4 minutes.

**In** conclusion, it was determined that while playing the reaction game the blind spot of the user is stimulated only 60% of the time due to the eye movements. There was a need to multiply the duration of gameplay by a factor of approximately 1/0.6 to obtain the effective stimulation time.

**Claims**

1.    A device (10) for selective application of stimulus light (66) to an optic nerve head (36) of one of a left eye (30) and a right eye (30) of a user, the device (10) comprising:

at least one light emitting source (60) configured to position emitted stimulus light (66) to impinge onto the optic nerve head (36) based on a determined location of the optic nerve head (36) with respect to the user's gaze (33);

at least one screen (50) configured to fixate the user's gaze (33) on a target area (52) of the screen (50) by engaging the user with content displayed on the at least one screen (50);

a processor (80) for selecting the stimulus light (66);

wherein the emitted stimulus light (66) is configured to stimulate melanopsin and is blue light;

wherein the at least one screen (50) is the light emitting source (60);

wherein the processor (80) comprises and executes software configured for positioning the blue stimulus light (66) on the screen (50) and for providing content displayed on the at least one screen (50), wherein the content is a game, and the game is displayed in the target area (52) of the screen.

2. The device (10) of claim 1, wherein the emitted stimulus light (66):

- flickers at a frequency in a frequency range between 6 and 20 Hz; and/or
- has an illuminance of more than 20 melanopic lux, preferably approximately 60 melanopic lux.

3. The device of one of claims 1 to 2, wherein the at least one light emitting source (60) is further configured to position the emitted stimulus light (66) to impinge on one of the left eye (30) and the right eye (30) of the user.

4. The device (10) of one of claims 1 to 3, wherein the at least one light source (60) is further configured to dimension the emitted stimulus light (66) to impinge on a portion of the optic nerve head (36) corresponding in size to 80% of the optic nerve head (36).

5. The device (10) of one of claims 1 to 4, wherein the at least one screen (50) is:

- arranged normal to the user's gaze (33); and/or
- arranged at a constant distance from the left eye (30) and the right eye (30).

6. The device (10) of one of claims 1 to 5, wherein the at least one screen (50) is configured to display the content within at least one target area (52) of the at least one screen (50), the at least one target area (52) corresponding to an area having a diameter of 1.0 to 5.0 degrees in a foveal region (39) of the left eye (30) and the right eye (30) when the gaze (33) is fixated on the at least one target area (52).

7. The device (10) of claim 6, wherein the at least one target area (52) is arranged at the center of the at least one screen (50) and optionally, wherein the at least one target area (52) is configured to fixate one of the left eye (30) and the right eye (30) of the user.

8. The device (10) of one of claims 1 to 7, wherein the device (10) is or comprises a smartphone (50, 60) or is a virtual reality headset, or wherein the device comprises a smart phone and a virtual reality headset, wherein the smart phone (50, 60) is insertable into the virtual reality headset.

9. The device (10) of claim 8, wherein the virtual reality headset comprises at least one lens for forming a two-lens system with at least one of the left eye (30) and the right eye (30) of the user.

10. The device (10) of one of claims 8 or 9, wherein the virtual reality headset comprises one optical path extending between the at least one screen (50) and the left eye (30) and comprises another optical path extending between the at least one screen (50) and the right eye (30).

11. The device (10) of one of claims 1 to 10, wherein the left eye (30) and the right eye (30) of the user are directed straight ahead when the user's gaze (33) is directed at the content with which the user is engaged.

12. The device (10) of one of claims 1 to 11, further comprising a game controller configured for the user to engage with the content displayed on the at least one screen (50).

13. The device (10) of claim 12, wherein the game controller is further configured to adjust a position (60x, 60y) of the stimulus light (66) within the screen (50) during calibration.

14. The device (10) of one of claim 1 to 13, further comprising a memory device configured to store data relating to the location of the optic nerve head (36), the data being obtained from one of user-controlled calibration, input into the device (10) of fundus image data, and population data.

**15.** The device (10) of one of claims 1 to 14, wherein the light emitting source (60) is configured to:

a. emit the stimulus light (66) for a stimulus duration of at least 1 minute and up to 20 minutes, preferably for between 8 and 10 minutes; and/or

b. emit the stimulus light (66) with one or more breaks in emission; preferably wherein the break is at least 15 seconds; and/or

c. emit the stimulus light (66) for at least 30 to 120 seconds before a break; and/or

d. emit the stimulus light (66) over a session of use of the device (10), wherein the duration of a session of use is at least 1 minute, and up to 30 minutes, or preferably 12 to 15 minutes, wherein a session of use is the time from turning on of the device to the turning off of the device.

**Patentansprüche**

**1.** Vorrichtung (10) zum selektiven Anlegen von Stimuluslicht (66) an einen Sehnervenkopf (36) eines linken Auges (30) oder eines rechten Auges (30) eines Benutzers, wobei die Vorrichtung (10) umfasst:

mindestens eine lichtemittierende Quelle (60), die so konfiguriert ist, dass sie das emittierte Stimuluslicht (66) so positioniert, dass es auf den Sehnervenkopf (36) auftrifft, basierend auf einer bestimmten Position des Sehnervenkopfes (36) in Bezug auf den Blick des Benutzers (33);

mindestens einen Bildschirm (50), der so konfiguriert ist, dass er den Blick des Benutzers (33) auf einen Zielbereich (52) des Bildschirms (50) fixiert, indem er den Benutzer mit einem auf dem mindestens einen Bildschirm (50) angezeigten Inhalt in Kontakt bringt;

einen Prozessor (80) zur Auswahl des Stimuluslichts (66);

wobei das emittierte Stimuluslicht (66) so konfiguriert ist, dass es Melanopsin stimuliert und blaues Licht ist;

wobei der mindestens eine Bildschirm (50) die lichtemittierende Quelle (60) ist;

wobei der Prozessor (80) Software umfasst und ausführt, die konfiguriert ist, um das blaue Stimuluslicht (66) auf dem Bildschirm (50) zu positionieren und um einen Inhalt bereitzustellen, der auf dem mindestens einen Bildschirm (50) angezeigt wird, wobei der Inhalt ein Spiel ist und das Spiel in dem Zielbereich (52) des Bildschirms angezeigt wird.

**2.** Vorrichtung (10) nach Anspruch 1, wobei das emittierte Stimuluslicht (66):

- flackert mit einer Frequenz in einem Bereich zwischen 6 und 20 Hz; und/oder
- eine Beleuchtungsstärke von mehr als 20 Melonpic Lux, vorzugsweise etwa 60 Melanopic Lux, aufweist.

**3.** Vorrichtung nach einem der Ansprüche 1 bis 2, wobei die mindestens eine lichtemittierende Quelle (60) ferner so konfiguriert ist, dass sie das emittierte Stimuluslicht (66) so positioniert, dass es entweder auf das linke Auge (30) oder das rechte Auge (30) des Benutzers trifft.

**4.** Vorrichtung (10) nach einem der Ansprüche 1 bis 3, wobei die mindestens eine Lichtquelle (60) ferner so konfiguriert ist, dass sie das emittierte Stimuluslicht (66) so dimensioniert, dass es auf einen Teil des Sehnervenkopfes (36) auftrifft, der in seiner Größe 80 % des Sehnervenkopfes (36) entspricht.

**5.** Vorrichtung (10) nach einem der Ansprüche 1 bis 4, wobei der mindestens eine Bildschirm (50) ein Bildschirm ist:

- der senkrecht zum Blick des Benutzers angeordnet ist (33); und/oder
- die in konstantem Abstand zum linken Auge (30) und zum rechten Auge (30) angeordnet sind

**6.** Vorrichtung (10) nach einem der Ansprüche 1 bis 5, wobei der mindestens eine Bildschirm (50) so konfiguriert ist, dass er den Inhalt innerhalb mindestens eines Zielbereichs (52) des mindestens einen Bildschirms (50) anzeigt, wobei der mindestens eine Zielbereich (52) einem Bereich mit einem Durchmesser von 1,0 bis 5,0 Grad in einem fovealen Bereich (39) des linken Auges (30) und des rechten Auges (30) entspricht, wenn der Blick (33) auf dem mindestens einen Zielbereich (52) fixiert ist.

7. Vorrichtung (10) nach Anspruch 6, wobei der mindestens eine Zielbereich (52) in der Mitte des mindestens einen Bildschirms (50) angeordnet ist und optional, wobei der mindestens eine Zielbereich (52) so konfiguriert ist, dass er entweder das linke Auge (30) oder das rechte Auge (30) des Benutzers fixiert.

8. Vorrichtung (10) nach einem der Ansprüche 1 bis 7, wobei die Vorrichtung (10) ein Smartphone (50, 60) oder ein Virtual-Reality-Headset ist oder umfasst, oder wobei die Vorrichtung ein Smartphone und ein Virtual-Reality-Headset umfasst, wobei das Smartphone (50, 60) in das Virtual-Reality-Headset einsetzbar ist.

9. Vorrichtung (10) nach Anspruch 8, wobei das Virtual-Reality-Headset mindestens eine Linse zur Bildung eines Zweilinsensystems mit mindestens dem linken Auge (30) oder dem rechten Auge (30) des Benutzers umfasst.

10. Vorrichtung (10) nach einem der Ansprüche 8 oder 9, wobei das Virtual-Reality-Headset einen optischen Pfad umfasst, der sich zwischen dem mindestens einen Bildschirm (50) und dem linken Auge (30) erstreckt, und einen anderen optischen Pfad umfasst, der sich zwischen dem mindestens einen Bildschirm (50) und dem rechten Auge (30) erstreckt.

11. Vorrichtung (10) nach einem der Ansprüche 1 bis 10, wobei das linke Auge (30) und das rechte Auge (30) des Benutzers geradeaus gerichtet sind, wenn der Blick (33) des Benutzers auf den Inhalt gerichtet ist, mit dem der Benutzer beschäftigt ist.

12. Die Vorrichtung (10) nach einem der Ansprüche 1 bis 11, die ferner eine Spielsteuerung umfasst, die so konfiguriert ist, dass der Benutzer mit dem auf dem mindestens einen Bildschirm (50) angezeigten Inhalt interagieren kann.

13. Vorrichtung (10) nach Anspruch 12, wobei die Spielsteuerung ferner so konfiguriert ist, dass sie während der Kalibrierung eine Position (60x, 60y) des Stimuluslichts (66) innerhalb des Bildschirms (50) einstellt.

14. Die Vorrichtung (10) nach einem der Ansprüche 1 bis 13, die ferner eine Speichervorrichtung umfasst, die so konfiguriert ist, dass sie Daten speichert, die sich auf die Position des Sehnervenkopfes (36) beziehen, wobei die Daten entweder aus einer benutzergesteuerten Kalibrierung, der Eingabe von Fundusbilddaten in die Vorrichtung (10) oder aus Bevölkerungsdaten stammen.

15. Vorrichtung (10) nach einem der Ansprüche 1 bis 14, wobei die lichtemittierende Quelle (60) so konfiguriert ist, dass sie:

   a. das Stimuluslicht (66) für eine Stimulusdauer von mindestens 1 Minute und bis zu 20 Minuten, vorzugsweise zwischen 8 und 10 Minuten, ausstrahlen; und/oder
   b. das Stimuluslicht (66) mit einer oder mehreren Emissionspausen emittieren, wobei die Pause vorzugsweise mindestens 15 Sekunden beträgt, und/oder
   c. das Stimuluslicht (66) mindestens 30 bis 120 Sekunden lang vor einer Pause ausstrahlen; und/oder
   d. das Stimuluslicht (66) über eine Benutzungssitzung der Vorrichtung (10) emittieren, wobei die Dauer einer Benutzungssitzung mindestens 1 Minute und bis zu 30 Minuten oder vorzugsweise 12 bis 15 Minuten beträgt, wobei eine Benutzungssitzung die Zeit vom Einschalten der Vorrichtung bis zum Ausschalten der Vorrichtung ist.


**Revendications**

1. Dispositif (10) destiné à appliquer de manière sélective une lumière de stimulation (66) à une tête de nerf optique (36) de l'un parmi l'œil gauche (30) et l'œil droit (30) d'un utilisateur, le dispositif (10) comprenant :

   au moins une source d'émission de lumière (60) configurée pour positionner la lumière de stimulation émise (66) de manière à frapper la tête de nerf optique (36) sur la base d'un emplacement déterminé de la tête de nerf optique (36) par rapport au regard fixe de l'utilisateur (33) ;
   au moins un écran (50) configuré pour fixer le regard fixe de l'utilisateur (33) sur une zone cible (52) de l'écran (50) de sorte que l'attention de l'utilisateur soit éveillée par le contenu affiché sur l'au moins un écran (50) ;
   un processeur (80) destiné à sélectionner la lumière de stimulation (66) ;
   la lumière de stimulation émise (66) étant configurée pour stimuler la mélanopsine et étant une lumière bleue ;
   l'au moins un écran (50) étant la source d'émission de lumière (60) ;
   le processeur (80) comprenant et exécutant un logiciel configuré pour positionner la lumière de stimulation bleue (66) sur l'écran (50) et pour fournir un contenu affiché sur l'au moins un écran (50), le contenu étant un jeu, et le jeu étant affiché dans la zone cible (52) de l'écran.

2. Dispositif (10) selon la revendication 1, la lumière de stimulation émise (66) :

   - scintillant à une fréquence dans une gamme de fréquences comprise entre 6 et 20 Hz ; et/ou
   - présentant un éclairement supérieur à 20 lux mélanopiques, de préférence d'environ 60 lux mélanopiques.

3. Dispositif selon l'une des revendications 1 à 2, l'au moins une source d'émission de lumière (60) étant en outre configurée pour positionner la lumière de stimulation émise (66) de manière à frapper l'un parmi l'œil gauche (30) et l'œil droit (30) de l'utilisateur.

4. Dispositif (10) selon l'une des revendications 1 à 3, l'au moins une source de lumière (60) est en outre configurée pour dimensionner la lumière de stimulation émise (66) afin de frapper une partie de la tête de nerf optique (36) correspondant en taille à 80 % de la tête de nerf optique (36).

5. Dispositif (10) selon l'une des revendications 1 à 4, l'au moins un écran (50) étant :

   - disposé normalement au regard fixe de l'utilisateur (33); et/ou
   - disposé à une distance constante de l'œil gauche (30) et de l'œil droit (30).

6. Dispositif (10) selon l'une des revendications 1 à 5, l'au moins un écran (50) étant configuré pour afficher le contenu dans au moins une zone cible (52) de l'au moins un écran (50), l'au moins une zone cible (52) correspondant à une zone ayant un diamètre de 1,0 à 5,0 degrés dans une région fovéale (39) de l'œil gauche (30) et de l'œil droit (30) lorsque le regard fixe (33) est fixé sur l'au moins une zone cible (52).

7. Dispositif (10) selon la revendication 6, l'au moins une zone cible (52) étant disposée au centre de l'au moins un écran (50) et, éventuellement, l'au moins une zone cible (52) étant configurée pour fixer l'au parmi l'œil gauche (30) et l'œil droit (30) de l'utilisateur.

8. Dispositif (10) selon l'une des revendications 1 à 7, le dispositif (10) étant ou comprenant un Smartphone (50, 60) ou étant un casque de réalité virtuelle, ou le dispositif comprenant un Smartphone et un casque de réalité virtuelle, le Smartphone (50, 60) pouvant être inséré dans le casque de réalité virtuelle.

9. Dispositif (10) selon la revendication 8, le casque de réalité virtuelle comprenant au moins une lentille destinée à former un système à deux lentilles avec l'un au moins parmi l'œil gauche (30) et l'oeil droit (30) de l'utilisateur.

10. Dispositif (10) selon l'une des revendications 8 ou 9, le casque de réalité virtuelle comprenant un chemin optique s'étendant entre l'au moins un écran (50) et l'œil gauche (30) et comprenant un autre chemin optique s'étendant entre l'au moins un écran (50) et l'œil droit (30).

11. Dispositif (10) selon l'une des revendications 1 à 10, l'œil gauche (30) et l'œil droit (30) de l'utilisateur étant dirigés droit devant lorsque le regard fixe de l'utilisateur (33) est dirigé vers le contenu auquel l'attention de l'utilisateur est éveillé.

12. Dispositif (10) selon l'une des revendications 1 à 11, comprenant en outre un contrôleur de jeu configuré pour que l'attention de l'utilisateur soit éveillé au contenu affiché sur l'au moins un écran (50).

13. Dispositif (10) selon la revendication 12, le contrôleur de jeu étant en outre configuré pour ajuster une position (60x, 60y) de la lumière de stimulation (66) dans l'écran (50) pendant l'étalonnage.

14. Dispositif (10) selon l'une des revendications 1 à 13, comprenant en outre un dispositif de mémorisation configuré pour stocker des données relatives à l'emplacement de la tête de nerf optique (36), les données étant obtenues à partir d'un étalonnage commandé par l'utilisateur, d'une entrée dans le dispositif (10) de données d'image de fond d'œil et de données de population.

15. Dispositif (10) selon l'une des revendications 1 à 14, la source d'émission de lumière (60) étant configurée pour :

   a. émettre la lumière de stimulation (66) pendant une durée de stimulation d'au moins 1 minute et jusqu'à 20 minutes, de préférence entre 8 et 10 minutes ; et/ou

b. émettre la lumière de stimulation (66) avec une ou plusieurs interruptions d'émission ; de préférence, l'interruption étant d'au moins 15 secondes ; et/ou

c. émettre la lumière de stimulation (66) pendant au moins 30 à 120 secondes avant une interruption ; et/ou

d. émettre la lumière de stimulation (66) pendant une session d'utilisation du dispositif (10), la durée d'une session d'utilisation étant d'au moins 1 minute, et allant jusqu'à 30 minutes, ou de préférence de 12 à 15 minutes, une session d'utilisation étant le temps écoulé entre la mise en marche du dispositif et l'arrêt de celui-ci.

*Fig. 1*

| |
|---|
| Locating optic nerve head |
| Positioning at least one light emitting source |
| Fixating user's gaze |
| Emitting stimulus light |

*Fig. 2*

*Fig. 3*

*Fig. 4*

*Fig. 5*

*Fig. 6*

Time after stimulation

10 min                                          20 min

**Fig. 7**

1 min

**Fig. 8**

## 10 min

**Fig. 9**

## b-wave

□ none   ■ 10 s   ▨ 1 min   ⊟ 10 min

**Fig. 10**

*Fig. 11*

*Fig. 12*

*Fig. 13*

**REFERENCES CITED IN THE DESCRIPTION**

**Patent documents cited in the description**

- WO 2016162554 A1
- WO 2010076706 A1
- WO 2014172641 A
- US 5923398 A
- US 20070182928 A
- WO 2016145064 A1
- WO 2018224671 A
- US 10444505 B
- EP 3281056 A1
- US 9283401 B